(19) Europäisches Patentamt
European Patent Office
Office européen des brevets

(11) **EP 3 245 028 B1**

(12) **EUROPEAN PATENT SPECIFICATION**

(45) Date of publication and mention
of the grant of the patent:
**10.08.2022 Bulletin 2022/32**

(21) Application number: **16780397.2**

(22) Date of filing: **12.01.2016**

(51) International Patent Classification (IPC):
***B25J 15/00*** *(2006.01)*

(52) Cooperative Patent Classification (CPC):
**B25J 15/0023; B25J 9/142; B25J 13/08;
G01L 1/246; G01T 7/00;** A61F 2002/7615

(86) International application number:
**PCT/US2016/013013**

(87) International publication number:
**WO 2016/167851 (20.10.2016 Gazette 2016/42)**

(54) **SOFT ROBOTIC DEVICE WITH FIBER BRAGG GRATING SENSOR**

WEICHE ROBOTISCHE VORRICHTUNG MIT FIBER BRAGG GRATING SENSOR

DISPOSITIF ROBOTIQUE SOUPLE AVEC CAPTEUR FIBRE BRAGG GRATING

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.01.2015 US 201562102363 P**

(43) Date of publication of application:
**22.11.2017 Bulletin 2017/47**

(73) Proprietor: **President and Fellows of Harvard
College
Cambridge, MA 02138 (US)**

(72) Inventors:
• **LESSING, Joshua, Aaron
Cambridge, MA 02141 (US)**
• **WHITESIDES, George, M.
Newton, MA 02458 (US)**
• **SHEVCHENKO, Yanina
Cambridge, MA 02140 (US)**
• **MOSADEGH, Bobak
New York, NY 10065 (US)**
• **GALLOWAY, Kevin, C.
Somerville, MA 02143 (US)**
• **TAYI, Alok, Suryavamsee
Somerville, MA 02143 (US)**

(74) Representative: **HGF
HGF Limited
1 City Walk
Leeds LS11 9DX (GB)**

(56) References cited:
US-A1- 2002 157 388     US-A1- 2009 137 952
US-A1- 2010 258 362     US-A1- 2012 271 339
US-A1- 2013 110 289     US-A1- 2014 109 560
US-A1- 2014 180 134

**EP 3 245 028 B1**

**Description**

TECHNICAL FIELD

This technology relates generally to soft robots or soft actuators that integrate sensors.

BACKGROUND

Soft devices are machines built from soft materials (e.g., elastomers, gels, liquids). These soft devices are useful for their ability to change their size and shape readily upon electrical, chemical, pneumatic, ferrofluidic, or hydraulic actuation. In addition, the low stiffness of the elastomeric materials used to construct these devices (Young's modulus < 10 MPa) enables them to deform readily in response to external forces. These attributes allow soft devices to perform functions that are challenging for hard machines. Examples include interacting with delicate, soft materials (e.g., biological tissues), and performing unstructured tasks (e.g., gripping objects of undefined shape). Machines, whether they are hard or soft, typically require the integration of electrical components (e.g. motors, sensors, microcontrollers, displays, pumps, batteries, etc.) in order to perform sophisticated tasks. These devices must be controlled in order to create an autonomous or semi-autonomous soft robotic system.

Knowing the morphology of a soft actuator is important for making a control system for a soft robot. This is because, unlike a hard robot, a soft robot can change volume and shape based on pneumatic or hydraulic inflation pressure or by forces in the external environment. In addition, unlike a hard robot, the response of the soft material of the actuator to force, whether external or internal, is highly non-linear making calculations that predict the behavior of the actuator in response to force very complex and difficult.

Having to know the morphology of the robot is an emergent problem that was not as prominent in the world of conventional hard robots. In a hard robot, force from the external environment generates a simpler outcome. For example, force applied to a hard robotic arm will move the arm a fixed distance that is easy to calculate since the robot is made from a series of hard components and linkages that do not deform during standard operation. In contrast, when force from the external environment is applied to a soft robotic arm, one gets a very complex outcome since the soft arm will both move and deform.

Additionally, the stiffness of the elastomer that makes up the actuator may change during actuation. For example, if the inflation pressure is at 30% of the max inflation pressure of an actuator, the elastomer is in a low strain state where the elastomer has stiffness "A"; and when the inflation pressure is at 80% of the max inflation pressure, the elastomer is in a higher strain state with a different stiffness "B". As a result, a different amount of force is required to achieve each increment of actuation.

Due to the intrinsic properties of elastomers, the stress vs. strain profile can be different for extension and relaxation. Elastomers show a high degree of hysteresis during cycles of loading and unloading. This discrepancy between the loading and unloading profile will change depending on how fast one cycles between the two. So as a result the system has memory. This aspect of elastomers will make soft actuators difficult to control using just the knowledge of the inflation pressure of the actuator. See, also, http://www.s-cool.co.uk/a-level/physics/stress-and-strain/revise-it/stress-strain-graphs.

The prior art documents US2012/0271339 A1 and US2014/0180134 A1 disclose balloon catheters comprising an elastomeric body having a chamber and a pressurizing inlet configured to receive fluid for the chamber, and further comprising a fiber Bragg grating-based optical sensor.

The prior art document US 3,981,528 discloses a robot finger comprising an elastomeric body having a chamber and a pressurizing inlet configured to recevie fluid for the chamber, and further comprising a strain limited layer disposed along one side of the elastomeric body.

The object of the present invention is to improve the control of a soft robotic device comprising an elastomeric body and a strain limited layer disposed along one side of the elastomeric body.

SUMMARY

In one aspect, the present invention provides a soft robotic device of claim 1. Soft robotic devices with a sensor or a network of sensors providing information about the state of the robot and/or its environments are provided. Non-limiting examples of such sensors include optical sensors, fiber-optics sensor, evanescent-wave sensor, plasmonic sensor, grating-based sensor, fluorescent sensors, and nonlinear optical sensors. These optical sensors may be implemented via a range of optical structures such as optical fibers, planar waveguides, custom made waveguides, and PCF. Other non-limiting examples include sensors capable of chemical, biological detection, sensors that can measure stress, direction of stress, sound. In still other embodiments, the sensors include thermal sensor, chemical sensor, biological analyte sensor, sound sensor, optical sensor, and radiological sensor. In certain embodiments, the sensor(s) are used

2

for the determination of the position, morphology, and/or physical state at points along the soft actuator or soft robot. The use of the sensor or network of sensors will allow for a real-time observation of the soft robotic device's current state, for example its three-dimensional position in space, velocity, acceleration, as well as sensing/perception of information about its environment, e.g., temperature, radiation, illumination, sound, presence of a certain chemical or biological agent. The feedback from the sensors can serve as inputs to a control system that determines the subsequent actions of the soft robotic device.

A soft robotic prosthetic system is also described, including a soft robot configured to assist the movement of one or more muscle or body part of a user and comprising an elastomeric body having one chamber or a plurality of interconnected chambers disposed within the body and a pressurizing inlet that is configured to receive fluid for the chamber or the plurality of interconnected chambers to actuate the soft robot; at least one sensor configured to detect physical, chemical, or electronic signal; and at least one of a processor configured to operably linked to the sensor to receive the readouts from the sensor and interpret the readouts; and a control system configured to actuate the soft robot to assist the movement of one or more muscle or body part of a user based on the readouts generated by the one or more sensors or the processor's interpretation of the readouts.

Soft robotic devices are also described having one or more imaging areas along the body of the soft robot for the determination of the position, velocity, acceleration, orientation, momentum and strain/morphology at points along the actuator visually, by motion capturing computer program or an x-ray imaging system. The imaging area may be a colored area with any recognizable color or a radiocontrast, e.g., a chemical which is recognizable via medical imaging.

A soft robotic device is also described, including: an elastomeric body having one chamber or a plurality of interconnected chambers disposed within the body and a pressurizing inlet that is configured to receive fluid for the chamber or the plurality of interconnected chambers; and at least one fiber Bragg grating-based optical sensor.

In any of the embodiments described herein, the grating-based sensor is configured to detect physical, chemical, biological, or electronic signal.

In any of the embodiments described herein, the grating-based sensor is selected from the group consisting of tilted fiber Bragg gratings sensor, chirped gratings sensor, and long period Bragg gratings sensor.

In any of the embodiments described herein, the grating-based sensor configured to provide information regarding the state of the soft robotic device.

In any of the embodiments described herein, the state of the soft robotic device is selected from the group consisting of the pressure, temperature, position, length, curvature, orientation, velocity, acceleration, morphology, stress, strain, and physical state at points along the soft robotic device.

In any of the embodiments described herein, the grating-based sensor configured to provide information regarding the external environment of the soft robotic device.

In any of the embodiments described herein, the grating-based sensor is configured to detect temperature, humidity, chemical agent or biological agent in the external environment of the soft robotic device; or the grating-based sensor is configured to detect strain, force, magnetic field, flow, bending, directional bending, three-dimensional state, vibration, pressure, temperature information of the soft robot.

In any of the embodiments described herein, the grating-based sensor is embedded in the elastomeric body or attached to the outside of the elastomeric body.

In any of the embodiments described herein, the grating-based sensor is molded or co-molded into the elastomeric body.

In any of the embodiments described herein, the grating-based sensor is sewn, glued, or snapped on to the elastomeric body or secured to the elastomeric body with hook and loop.

In any of the embodiments described herein, the grating-based sensor is removable from the elastomeric body.

In any of the embodiments described herein, the grating-based sensor helically winds around the elastomeric body or part thereof.

In any of the embodiments described herein, the soft robotic device comprises one or more grating-based sensor embedded in or attached to the strain limited layer.

In any of the embodiments described herein, the soft robotic device further comprises a plurality of spectrally separated grating-based sensors with different periods.

In any of the embodiments described herein, the plurality of spectrally separated grating-based sensors with different periods are disposed together along the length of a single piece of fiber or disposed individually and spliced together.

In any of the embodiments described herein, the pressurizing inlet that is configured to receive fluid from an external fluid source.

In any of the embodiments described herein, the soft robotic device comprises one or more grating-based sensors embedded in or attached to the strain limited layer and one or more grating-based sensors embedded in or attached to the elastomeric body.

In any of the embodiments described herein, the soft robotic device further comprises one or more additional sensors each independently selected from the group consisting of grating-based sensor, biological analyte sensor, sound sensor, optical sensor, radiological sensor, thermal sensors, strain sensors, chemical sensors, biological sensors, neural sensors, pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, chemical weapons sensors, VX gas sensors, sarin gas sensors, mustard gas sensors, explosives detectors, metal detectors, and current sensors.

In any of the embodiments described herein, the soft robotic device further includes at least one of a processor configured to operably linked to the grating-based sensor to receive the readouts from the grating-based sensor and interpret the readouts; and a control system configured to control the movement of the soft robot based on the readouts generated by the grating-based sensor or the processor's interpretation of the readouts.

As used herein, the term "soft robotic device" refers to a soft robot or a soft actuator. As used herein, the term "strain limited layer" and "strain limiting layer" are used interchangeably. Strain is a description of deformation in terms of relative displacement of a body. A deformation results from a stress induced by applied forces, in the case here, for example, by the pressurizing force. Because materials of lower stiffness or smaller elastic modulus will deform to a greater degree than the higher elastic modulus materials, the low stiffness materials experience more strain or deformation. As a result, the strain in the material of higher stiffness or greater elastic modulus is smaller or "limited." As used herein, the layer or wall or portion thereof of the soft robot that extends, bends, expands or unfolds at lower threshold force is the 'extensible' or 'low strain' member. The layer or wall or portion thereof of the soft robot that extends, bends, expands or unfolds at higher threshold force is referred herein as the "strain limited" layer or wall or membrane.

In certain embodiments, the term "strain limiting layer" refers to a layer which is stiffer or less stretchable than the elastomeric body and is attached or secured to the elastomeric body. In one or more embodiments, the strain limited layer is more than about 10%, 20%, >50%, >100%, or >500% stiffer than the elastomeric body.

As used herein, the term "state" of the soft robot refers to the general operation status of the soft robot. The state of a soft robot or its system is described by a set of state variables. The state variables of a system are any set of measurable quantities that together provide enough information about the system to describe the present and/or future behavior of a robot to a user; or set of variables that the user desires to observe. A sufficient set of state variables can consist of a single measurable quantity or a set of measurable quantities depending on the system and what the user wishes to observe. The criteria for defining a set of state variables as sufficient is that the set provides enough information to accurately predict or approximate the present and/or future behavior of a measurable quantity or set of measurable quantities the user desires to observe. Non-limiting examples of state variables for a soft robot include the robot's position, the robot's orientation, the robot's velocity, the robot's acceleration, the elapsed time since the robot was last actuated, the maximum pressure of the pressurizing fluid used during the robots last actuation, the volume of pressurizing fluid in an actuator, the surface curvature of an actuator, material stress at points along the body of the robot, material strain at points along the body of the robot, the force being applied by the robot on an object, the robots temperature, the pressure inside of an actuator, the pressure outside of an actuator, the pressure differential between the pressurizing fluid inside of an actuator and the ambient pressure in the actuators external environment.

BRIEF DESCRIPTION OF THE FIGURES

The following images also describe details for multiple applications and features that can be incorporated into the structures. In these examples, we assume there is a connection in the soft robotic device to a pressurized fluid source. The invention is described with reference to the following figures, which are presented for the purpose of illustration only and are not intended to be limiting. In the Drawings:

**Figure 1** is a schematic diagram of a fiber Bragg grating.

**Figure 2** is a schematic diagram of a tilted fiber Bragg grating.

**Figure 3** is a schematic diagram that shows several TFBGs with different periods imprinted in the core of the same fiber.

**Figure 4A** is a schematic diagram showing a soft tentacle with an optical waveguide embedded in its structure.

**Figure 4B** is a schematic diagram that shows a chirped grating imprinted in the core of an optical fiber.

**Figure 4C** illustrates a chirped grating integrated into a tentacle arm whereby the sensor can provide state feedback of the arm shape as well as the end effector shape.

**Figure 5** illustrates a GBS integrated into a tentacle arm whereby the sensor can provide state feedback of the arm shape as well as the end effector shape.

**Figure 6A** is another embodiment of the grating-based sensor in which the sensor is helically wound around the soft device.

**Figure 6B** illustrates a 3D mapping of the state of the soft device as measured by the grating-based sensor.

**Figures 7A-7B** are schematics of a soft actuator regulated by a pneumatic controller that inflates (Fig. 7B) or deflates (Fig. 7A) based on signals from a microprocessor, based on signals from an external sensor connected to a human or animal.

**Figures 8A-8C** show the electrically-mediated signaling for soft actuators. **Figure 8A**): Movement of an arm stretches an elastic band with an embedded conductive material that changes resistance upon stretching. **Figure 8B**): Electrodes attached to a muscle measuring fluctuations in voltage potential when the muscle is contracted. **Figure 8C**): Electrodes attached to the head measuring fluctuations in voltage potential when neurons are triggered.

**Figure 9** shows a soft actuator with colored marks on its straining surfaces.

**Figure 10** shows a soft actuator with colored marks on its strain limiting surfaces.

**Figure 11A** shows the schematic view of a soft robotic device including a plurality of radiological sensors. **Figure 11B** shows the schematic view of a soft robotic device including a plurality of radiological sensors and in its actuated state detecting radioactive material.

**Figure 12A** shows the schematic view of a soft robotic device including a plurality of scintillating sensors detecting radioactive material. **Figure 12B** shows the schematic view of a soft robotic device including a plurality of scintillating sensors detecting radioactive material.

**Figure 13** shows the schematic view of a soft robotic device including a plurality of chemical sensors detecting a chemical source.

**Figure 14** shows the schematic view of a soft robotic device including a plurality of optical sensors detecting an illumination source.

**Figure 15A** shows the schematic view of a soft robotic device including a plurality of thermal sensors. **Figure 15B** shows the schematic view of a soft robotic device including a plurality of thermal sensors detecting the thermal environment.

**Figure 16** shows the schematic view of a soft robotic device including a plurality of sound sensors detecting the acoustic environment.

DETAILED DESCRIPTION

A soft robotic device having one or more sensor(s) or imaging areas integrated, embedded, attached, or otherwise linked or connected to the soft robotic device is described. In some embodiments, a soft robot is described, including an elastomeric body having one chamber or a plurality of interconnected chambers disposed within the body, the elastomeric body comprising a pressurizing inlet that is configured to receive fluid into the chamber or the plurality of interconnected chambers from a fluid source; and a strain limited layer disposed along one side of the elastomeric body; and at least one sensor or imaging area. In certain embodiments, the sensor is configured to detect a physical, chemical, and/or electronic signal and/or to provide state estimation of the soft robot. In certain embodiments, the one or more sensors are embedded, integrated, attached, or otherwise linked or connected to elastomeric body. In certain embodiments, the one or more sensors are embedded, integrated, attached, or otherwise linked or connected to the strain limited layer. In certain embodiments, one or more sensors are embedded, integrated, attached, or otherwise linked or connected to the strain limited layer and one or more other sensors is embedded, integrated, attached, or otherwise linked or connected to the elastomeric body. In certain embodiments, one or more sensors are external to the strain limited layer or the elastomeric body.

In some embodiments, the sensors may be used to provide estimation of the state of the soft robotic device. The state may be selected from the group consisting of the pressure, position, length, curvature, orientation, velocity, acceleration, strain, stress, morphology, and physical state of the soft robotic device. In certain embodiments, a user or a processor can process the readout from the sensor to determine the strain state of the soft actuator given the material properties of the soft actuator and the strain data from the strain sensors. Thus, one can collect the stress vs strain profile for a test sample of elastomer. The resulting data set can be used to create a look up table that correlates the relationship between the measured strain at a point on the actuator and the corresponding material stress at that point on the actuator.

In certain embodiments, the sensor is one or more sensors selected from the group consisting of flow sensors, volume detection system or volume sensors, grating-based sensors, sound sensor, optical sensor, radiological sensor, thermal sensors, strain sensors, chemical sensors, biological sensors, neural sensors, pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, VX gas sensors, sarin gas sensors, mustard gas sensors, explosives detectors, metal detectors, radiological detectors, and current sensors.

In certain embodiments, the soft robot described herein includes more than one type of sensors. In certain embodiments, the soft robot described herein includes two or more types of sensors each selected from the group consisting of grating-based sensors, sound sensor, optical sensor, radiological sensor, thermal sensors, strain sensors, chemical sensors, biological sensors, neural sensors, pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, chemical weapon sensors, VX gas sensors, sarin gas sensors, mustard gas sensors, explosives detectors, metal detectors, radiological detectors, and current sensors. The use of more than one type of sensors in a soft robot will provide rich information (e.g., curvature, position or location) regarding the status of the soft robot.

In some embodiments, the sensors, sensor networks, or sensor systems typically are flexible and compliant, and capable of large deformation of equal or greater range than the soft actuator itself.

The soft robot can be any robot having an expandable body that is capable of expansion or collapse on change of pressure. In some embodiments, the soft body of the soft robotic device has a pressurizing inlet that is configured to communicate with a fluid source, an expandable body and a strain limited layer secured to a portion of the expandable body. The examples of the actual construction of the soft robot are non-limiting and the expandable body can be, for example, made from a plurality of expandable fluidly interconnected chambers; where the pressurizing inlet is configured to communicate with the plurality of expandable interconnected chambers, or made using one or more elastomeric chambers configured to expand upon fluidic pressurization and/or contract upon vacuum actuation. In other embodiments, the expandable body is made from one or more flexible or extensible chambers configured to unbend or unfold upon fluidic pressurization. The soft body robotic device further includes a strain limited layer, which is inflexible or less flexible than the elastomeric body, attached to the elastomeric body. In one or more embodiments, the strain limited layer is inextensible, or the strain limited layer can accommodate strain of less than 35% or less than 40% or less than 50%, and for example can be in the range of 0-50% strain. The elastomeric body in the soft body robotic device can be configured to preferentially expand when the chamber or the plurality of interconnected chambers are pressurized by the fluid, causing a bending motion around the strain limiting layer. In other embodiments, a strain limited layer is wrapped around the body in a helix to form a twisting actuator. *See,* WO 2012/148472; International Application No. PCT/US13/28250 filed February 28, 2013; International Application No. PCT/US 13/22593 filed January 22, 2013 and US Provisional application Serial No. 61/885092, filed October 1, 2013, for non-limiting description of soft actuators suitable for use in the current invention, the contents of which are incorporated by reference.

In certain embodiments, the soft robot further includes a control system for controlling the motion of the soft robot based at least in part on data obtained from one or more sensors or the imaging areas.

One important application for performing real time measurements of a soft device's morphology is to compensate for hysteresis in the inflation behavior of the device. For example when a soft actuator is inflated to a given pressure Y followed by being inflated to a new pressure X, where X > Y, and then inflated again to a pressure of Y, it is sometimes observed, depending on conditions, that a larger degree of actuation occurs on the second inflation to Y. For systems where this hysteresis effect is prominent, knowing the pressure supplied to a soft device is insufficient for determining its morphology. In these cases, a network of sensors or markers (e.g. strain sensors, magnetic markers, LED markers, etc.) that aid in the measurement of parameters that are independent of pressure could be used to determine the morphology of a soft actuator or robot. Such a system of sensors could be used to guarantee that the desired morphology of a soft device is achieved regardless of the device's memory of past inflations.

*Volume Detection System and Pressure Sensors*

In some embodiments, a soft robot or soft actuator with one or more volume sensors, volume detection system (e.g., flow sensors), or pressure sensors is described. Volume detection system (e.g., flow sensors), or pressure sensors each may be embedded in the chamber of the soft robot or soft actuator and are each configured to measure the volume of the fluid flowing into or out of the chamber or the pressure inside the chamber. As used herein, the term "volume detection system" generally refers to any sensor or system which is configured to determine the volume of fluid in the chamber(s) of the soft actuator. A specific example of the volume detection system is a flow sensor.

Thus, in some embodiments, the flow sensor is configured to measure air flow into the soft robot or soft actuator. In other embodiments, the flow sensor is configured to measure air flow out of the soft robot or soft actuator. In still other embodiments, a single flow sensor or a series of flow sensors are each used to measure air flow both in and out of the soft robot or soft actuator. In other embodiments, the soft robot or soft actuator is part of a soft robotic system which comprises at least one of a processor and a control system. The processor is configured to receive the data readout from the volumetric or pressure sensor. Based on the interpretation of the readout, the processor may send instructions to the control system to reduce or stop more volume of the fluid from going into the chamber or adjust the pressure inside the chamber. Therefore, the readout from the volumetric or pressure sensors may serve as an indicator for the inflation state of the chamber of the soft robot.

In certain embodiments, the soft robot comprises one or more valves controlling the flow of the fluid into and out of the chamber(s). In some embodiments, the valve is closed after the soft robot's chambers are inflated with the fluid with a desirable volume and/or pressure. In these embodiments, when the soft robot encounters an obstruction in its path *(e.g.,* a soft robotic gripper colliding with an object), the pressure inside the chamber of the soft robot may rise or oscillate due to compression of the soft actuators. For these embodiments the collision could be detected with a pressure sensor that senses the rise or oscillation in pressure due to compression of the actuators upon collision.

In other embodiments, the valve remains open during operation and the desired pressure in the actuator is maintained by a controller system (e.g., an electrical or mechanical pneumatic regulator). In some embodiments, when the soft robot encounters an obstruction in its path *(e.g.,* a soft robotic gripper gripping an object), the controller system would stop providing pressurizing fluid to the chamber(s) of the actuator(s) upon colliding with the object since supplying additional fluid to an actuator whose actuation path is obstructed would require increasing the internal pressure in the actuator above the set pressure maintained by the controller system. After the desirable pressure is reached, the controller system may close the valve.

In certain embodiments, a fluid, *e.g.,* air, is supplied to a soft actuator via a pressure regulator (mechanical or electrical) then the actuator will be able to maintain a fixed pressure regardless of its interactions with its environment. In these embodiments, the volume of air inside the actuator may change depending on whether external forces are applied on the actuator. As a result, in certain embodiments, a volume detection system can be used to measure air flow in and out of an actuator to determine whether there has been a change in state of the actuator. For example, if a soft actuator is inflated and its actuation path is unobstructed, it will inflate to its full volume (which can be measured by a volume detection system) at that pressure.

If a force is applied on the actuator *(e.g.,* due to obstruction), some of the air in the actuator could flow out of the actuator as the actuator is deformed. Thus, in some specific embodiment, a change in the volume inside the actuator measured by the volume detection system is used for detecting/sensing collision of the soft robot with an object. In some embodiments, the soft actuator is inflated and its actuation path is obstructed, e.g., grasping an object, the actuator would not inflate to its full volume because it would stop inflating shortly after it hits that object. In still other embodiments, a soft actuator in a soft gripper grips an object which may fall out of its grasp. The actuators may start to take in air and inflate to its full volume because once the object is lost the actuator, e.g., the gripper, is unobstructed.

In still other embodiments, the flow sensor is configured to determine/estimate the size of the object gripped. In certain embodiments a soft robotic gripper would be used to grip objects of varying size. If the object being gripped is small the actuators will need to take on a large volume of air to nearly close the gripper all the way. If the object is large only a small amount of fluid *(e.g.,* air) will need to be provided since the gripper will not need to close to a large degree before it contacts the object to form a grip. By measuring the amount of fluid required to grip an object it is possible to estimate the objects size.

In still other embodiments, the inflation profile (e.g., the change in fluid volume inside the actuator as a function of time measured by the flow sensor) of an actuator can be used to measure the compliance of an object. The object may have a soft body *(e.g.,* a sponge) and easily comply with the gripping force of the actuator. In this case, the actuator will fill with air quickly until the actuator's gripping path is obstructed by colliding with the sponge. Then the actuator will start to fill with air slowly as it slowly compresses the sponge. One may measure the change of the rate of fluid *(e.g.,* air) flow into the soft robot or soft actuator to determine/estimate when the robot has gripped the object, *i.e.,* the measurement of the time-dependent change of the pressure and/volume. When the object is fragile, a user or a control system may stop the supply of air to the gripper to prevent further deformation of the object. Thus, the volumetric sensor can provide information to enable a user to prevent a soft gripper from damaging an object via over compression. Thus, in some embodiments, the soft robot device comprising a volumetric sensor further includes a processor and/or a controller system with instructions embedded in the processor or controller system to instruct the control system to begin a corrective action if the volumetric sensor detects a fluid volume inside the chamber to be over a threshold value. In other embodiments, the volumetric sensor further includes a processor and/or a controller system with instructions embedded in the processor or controller system to instruct the control system to begin a corrective action if it measures an anomalous change in the rate of fluid flow into or out of an actuator that corresponds to crushing an object.

In some embodiments, the soft robotic device (e.g., a gripper) is configured to respond to anomalous pressure or flow readings, *e.g.,* to identify and correct for patterns in the time dependent flow or pressure response that correspond to undesired events. In some embodiments, the soft robotic device *(e.g.,* a gripper) further includes a processor and/or a controller system configured to detect time-dependent flow and/or pressure change. In certain embodiments, the pressure in the actuators of a soft robotic gripper is maintained by a regulator and the processor and/or a controller system is configured to detect a sudden increase or oscillation of flow and/or a sudden decrease or oscillation in pressure (e.g., when the gripper drops the gripped object by accident) and to instruct the controller system to attempt to grip the object again. In other embodiments, the processor and/or a controller system is configured to detect a flow profile characterized by a sudden decrease of flow followed by a continuous and slower rate of flow of the fluid into the chamber(s) and to instruct the controller system to stop further fluid from flowing into the chamber(s). For instance, this may occur after gripping is detected (which would be seen as a sudden decrease in flow) followed by compressing the gripped object (which would be seen as a slower rate of flow corresponding to the slow compression of the grasp target). When this characteristic pattern of flow is detected, the processor or the controller system could elect to stop providing fluid *(e.g.,* air) to the actuator's chamber(s) in order to stop the continued compression of the gripped object.

In some embodiments, the soft robotic device, *e.g.,* a soft gripper, includes a force sensor used for state estimation of the soft device/robot. As described herein, if a soft actuator is making contact with an object, knowing the pressure and the volume of air used to inflate the actuator may not be enough information to know the actuators morphology. In this case one could use data on the inflation pressure and the volume of air used to inflate the actuator in conjunction with readings from force sensors on the surface of the actuator to determine the actuators morphology. This combination of pressure, air flow, and force information will be important for controlling a soft robotic gripper.

### *Optical sensors for soft actuators based on fiber Bragg gratings*

In one aspect, a soft robotic device is described, including: an elastomeric body having one chamber or a plurality of interconnected chambers disposed within the body and a pressurizing inlet that is configured to receive fluid for the chamber or the plurality of interconnected chambers; and at least one grating-based sensor. In some embodiments, the grating-based sensor is configured to detect physical, chemical, and/or electronic signal.

Sensing can be implemented with a variety of different optical sensors. As used herein, grating-based sensor refers to a sensor made by including/embedding a grating-like structure in an optical waveguide. An optical waveguide is a structure, such as a fiber or optical slab waveguide that guides the propagation of light. Guiding of light is possible because of the refractive index mismatch between the waveguide and ambient, or because of the refractive index mismatch between different layers of the waveguide, e.g., the core and the cladding. Examples of optical waveguides include planar waveguides, ridge waveguides, circular, channel and photonics crystal fiber waveguides. Typical optical waveguides are optical fibers having an inner core (typically of higher refractive index) and an outer cladding surrounding the core. The grating-based sensor can be an integral component of the waveguide located in the core and/or the cladding layers.

In certain embodiments, the soft robotic device further includes at least one of a processor configured to be operably linked to the grating-based sensor to receive the readouts from the grating-based sensor and interpret the readouts; and a control system configured to control the movement of the soft robot based on the readouts generated by the grating-based sensor or the processor's interpretation of the readouts. In certain embodiments, the phrase "grating based sensor" refers to an optical fiber. In other embodiments, the phrase "grating based sensor" refers to a system including the full set of items required to sense a signal, which includes 1) a light source that is feed into the fiber, 2) the fiber Bragg grating, and 3) a spectrometer to measure the light coming out of the fiber; and/or 4) an analysis software to interpret the measured spectrum. Thus, in certain embodiments, the soft robotic devices include a spectrometer to read the grating based sensor's readouts and then the processor may interpret the results from the spectrometer.

In some embodiments, the grating based sensor is a fiber Bragg grating-based optical sensor. In one or more embodiments, the fiber Bragg grating is integrated into an optical fiber. The optical fiber can be made of glass, polymers or glass/polymer hybrids, any kind of light transmitting material. Non-limiting examples of the grating-based sensors include fiber Bragg gratings, tilted fiber Bragg gratings (TFBG), chirped gratings sensors, and long period Bragg gratings sensors.

A fiber Bragg grating is a periodic optical element imprinted in a fiber's core. It works by reflecting back light whose wavelength is proportional to the period of the grating. The relationship between the period of the grating and its Bragg wavelength can be described in the following way:

$$\lambda B = 2\eta eff \Lambda, \qquad (1)$$

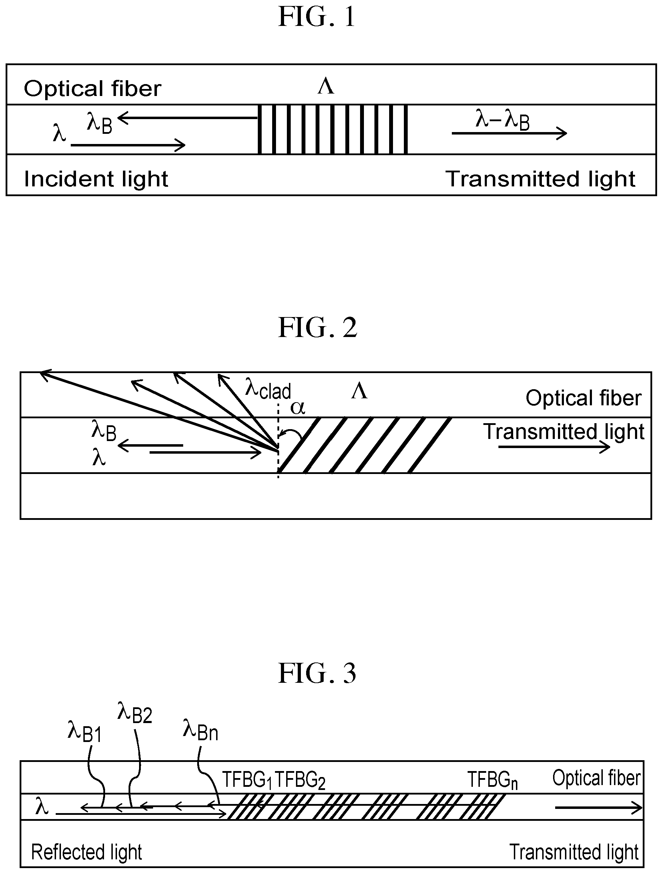

where A is the period of the grating, $\lambda B$ is the wavelength of the back-reflected light, and $\eta eff$ is the effective index of the back-reflected core mode [equation 1]. **Figure 1** shows a schematic diagram of a fiber Bragg grating. The structure of the FBG can vary via the refractive index, or the grating period. The grating period can be uniform or graded, and either localized or distributed in the grating. Fiber Bragg gratings can be made by "inscribing" or "writing" systematic (periodic or aperiodic) variation of refractive index into the core of an optical fiber using established techniques.

Imprinting of the Bragg grating at an angle with respect to the normal to the direction of the propagation of light in a fiber forms a tilted Bragg grating (TFBG). Back-reflected light consists of several frequencies, and it includes the back-reflected core mode and a group of back-propagating cladding modes. Wavelengths and effective indexes of excited modes can be found using the following relation:

$$\lambda clad = (\eta eff, core + neff, clad)\Lambda/\cos(\alpha), (2)$$

where $\lambda clad$ is the wavelength of the back-reflected cladding mode, $\eta eff, core$ is the effective index of the back-reflected cladding mode, $\eta eff, clad$ is the effective index of the back-reflected core mode, A is the period of the grating, $\alpha$ is the tilt of the grating. **Figure 2** shows a schematic diagram of a TFBG.

The fiber Bragg gratings can be used to sense a wide array of parameters (e.g., strain, bending, directional bending, the 3D state of the fiber, vibration, pressure, temperature, humidity, presence and concentration of a chemical or biological agent). A number of fiber Bragg gratings can be integrated into a single optical fiber to allow the sensors to be multiplexed within the same optical waveguide, therefore enabling continuous monitoring of the parameter of interest along the length of the waveguide. Information that can be obtained from the sensors can be used to derive information regarding the state of the soft robotic device such as the pressure, position, length, curvature, orientation, velocity, acceleration, morphology, and physical state at points along the soft robotic device. In other embodiments, the grating-based sensor can be configured to provide information regarding the external environment of the soft robotic device, such as temperature, humidity, chemical agent or biological agent in the external environment of the soft robotic device; or the grating-based sensor is configured to detect strain, force, magnetic field, flow, bending, directional bending, three-dimensional state, vibration, pressure, temperature information of the soft robot.

The grating-based sensors may be connected, linked, or attached to the soft robotic device in a variety of ways. In some embodiments, the grating-based sensor is embedded in the elastomeric body or attached to the outside of the elastomeric body. In other embodiments, the grating-based sensor is molded or co-molded into the elastomeric body. In still other embodiments, the grating-based sensor is sewn or snapped on to the elastomeric body or secured to the elastomeric body with hook and loop. The grating-based sensor may be permanently attached to or embedded in the elastomeric body or removable from the elastomeric body. The grating-based sensor may helically wind around the elastomeric body and/or the end-effector of the elastomeric body. In certain embodiments, the soft robotic device further comprises a strain limited layer disposed along one side of the elastomeric body; and the soft robotic device comprises one or more grating-based sensor embedded in or attached to the strain limited layer.

Sensing mechanism:

Tilt of the grating enables coupling of the core mode into back-propagating core and cladding modes. While a core mode can only propagate in the core of the fiber, cladding modes propagate in the cladding, they can be close to the surface of the fiber. This fact explains their sensitivity towards a range of external parameters including chemical changes. Temperature sensitivity of a grating can be explained by the fact that the core of the fiber will change its dimensions if the temperature changes. As a result the period of the grating will also change. Therefore straight and tilted Bragg gratings can be used for temperature monitoring.

A Bragg grating embedded in a soft robot can provide the robot with a structural sensing capability. Tilted fiber Bragg grating sensors can be used to monitor such parameters as vibration, strain, bending and directional bending. Change in any of the mentioned above parameters may cause change in the fiber's geometry - geometry of the core and cladding parts of the fiber. Tilted fiber Bragg gratings allow for spectrally-encoded monitoring of the structural changes through generation of a set of back-propagating cladding modes. Transmitted spectrum of a TFBG has a multiple of deeps that correspond to the back-reflected code and cladding modes. Each deep has a specific wavelength and amplitude. Monitoring of the position of the back-reflected modes or their amplitude allows for a continuous tracking of factors generating these changes, including structural changes.

In addition, tilted fiber Bragg grating sensors can be used to discriminate between several parameters such as temperature and strain, or strain and vibration. Discrimination between the parameters is possible because of the effect these parameters have on the back-reflected modes. For example, a change in the temperature affects all of the back-propagating modes in the same way. As a results wavelength position of the back-reflected modes is going to change by the same amount if temperature changes. Strain, on another hand, selectively affects only modes whose propagation is interfered by the applied force. As a result, only a few cladding modes are going to go through a transformation when strain is applied. Discrimination between temperature and strain is possible to implement by tracking relative change of the amplitude and wavelength of the selected resonances with respect to each other.

Propagation of the cladding modes close to the surface of the fiber enables dependency of their effective indexes on the refractive index of the exterior. Tilted fiber Bragg grating sensors can be successfully applied towards monitoring of different chemical and biological parameters. In certain embodiments, the fiber Bragg grating sensors described herein are used for humidity sensing, chemical sensing, biosensing and cellular sensing. In certain embodiments, chemical and/or biological sensing is implemented by adding a fiber Bragg grating sensor to the surface of the soft robot. In certain embodiments, sensors are partially embedded into the body of the soft robot or sealed to the surface of the robot using an adhesive. In these embodiments, adhesive may not limit flexibility of the optical waveguide. Flexible nature of existing commercial fiber and custom-made waveguides should allow for a successful application of sensors in combination with soft robots - sensors can be bent and can change their shape without degrading their performance.

Long period Bragg gratings are Bragg gratings with a period above 100 $\mu$m. Long period Bragg gratings function similarly to tilted fiber Bragg grating sensors, except that they can only excite forward propagating cladding and core modes. In certain embodiments, long period Bragg grating sensors are applied towards chemical, humidity, biological, pressure and bending sensing. Long period Bragg gratings, similarly to tilted fiber Bragg grating sensors, can be spectrally separated by having different periods and multiplexed in the same optical waveguide in order to achieve continuous monitoring of a parameter of interest along the length of the fiber.

In certain embodiments, long period grating sensors are integrated into soft robots either by adding them to the surface of the robots using an adhesive or by embedding them into the body of the soft robot, depending on the application. In certain embodiments, for chemical and biological sensing, the sensors are positioned on the surface of the robots, and part of the fiber's surface needs to be exposed to the ambient and interact with biological or chemical agents of interest. In certain embodiments, structural sensing can be implemented by sensors that are embedded into the soft robots or on the surface of soft robots. In certain embodiments, embedding is performed either by adding sensors to the pre-solidified polymer during the molding process or by positioning them in between soft layers when a robot is put together. In some embodiments, a sensor may be added to a strain-limiting layer that would not go through a significant stretching during the robot's actuation. In certain embodiments, the sensor is added to a regular soft layer that stretches upon actuation, and thus the optical waveguide is to be twisted, bent or spatially modified in a way that would permit stretching of the whole structure.

In some embodiments, the fiber could be embedded in the elastomer in a serpentine or helical pattern so that it can unfurl upon the stretching of the actuator. In this way a fiber could be placed into a stretching region of the actuator. In certain embodiments, the fiber is embedded in a straight-line geometry in the strain limiting layer.

**Figure 3** shows a schematic diagram that shows several tilted fiber Bragg grating sensors with different periods imprinted in the core of the same fiber.

In some embodiments, in order to arrange for several sensors to be imprinted in the same waveguide and be able to monitor a parameter of interest (e.g., gradient or bending) along a certain length of the fiber, the gratings need to be spectrally separated. This can be accomplished by making sure that period of each grating is different from periods of other gratings. By fabricating gratings with their individual periods and connecting them together it is possible to create a distributed network of sensors that could be used for monitoring of a parameter of interest along the length of the fiber. Responses of the sensors would not overlap due to spectral separation of the gratings. Each of the sensors would have their own spectral width that could be determined by the tilt of the grating.

Connection of several gratings of different periods within the same fiber can be implemented either by writing several gratings along the length of a single piece of fiber or by writing gratings in individual fiber pieces and later splicing these pieces together.

Chirped gratings or aperiodic fiber gratings are gratings that have a period that varies along the length of the grating.

Chirped gratings can be used for the implementation of a distributed structural or distributed chemical sensing, as an alternative to multiplexing of non-chirped gratings.

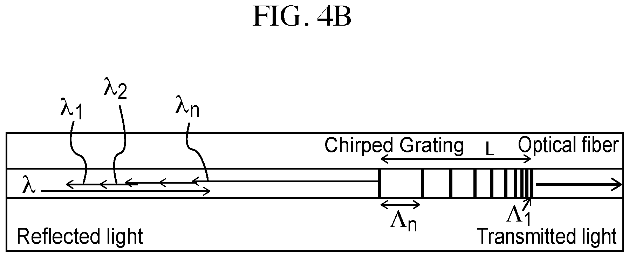

In some embodiments, Figure 4B shows a straight chirped Bragg grating of a length L. Period of the grating increases from A1 to An. A period that varies along the length of the grating generates a spectral response that is spatially encoded. Particularly, such grating's response will have resonances that are generated by specific parts of the grating of a specific periodicity. The wavelength of the back-reflected resonances is going to be dictated by the local period of the grating. Those resonances could be used to track structural and chemical changes happening along the length of the grating.

Specifically, when there is a grating in an optical fiber, it will back reflect (*i.e.*, mirror backward) certain light determined by the period of the grating and the effective refractive index of the grating. In the case of a multiplexed grating, one would expect to have more than one grating region in the fiber, each with a different periodicity, so that if light of multiple wavelengths is sent through the fiber, the individual wave lengths will be back-reflected when they hit a grating of appropriate periodicity. For example, if light of wavelength $\lambda BX$ and wave length $\lambda BY$ is sent through a fiber, the light of wavelength $\lambda BX$ will be back reflected when it hits a grating of periodicity A = $\lambda BX/2\eta eff$ and the light of wave length $\lambda BY$ will be back reflected when it hits a grating of periodicity A = $\lambda BY/2\eta eff.$ Thus, if these two distinct gratings are in different locations along the length of the fiber, one may use the grating-based sensor to provide information at these two separate locations.

To use distinct gratings may mean that one needs to write or splice a grating of unique periodicity into the fiber for every point for information-gathering. In certain embodiments, a chirped grating is used to solve this problem. For a chirped grating, the periodicity of the grating is gradually changing along its length. As a result, the wavelength being back-reflected is gradually changing along its length. This means that the chirped grating provides spatially resolved information at every point along the grating.

In some embodiments, each back reflection provides information about the local environment and the state of the grating at the point in the fiber where the back reflection occurred. This is because any disturbance to the core of the fiber, its cladding, or to the index of the surrounding environment can alter the behavior of the grating which alters the properties of the back reflected light. For example, if a grating is heated it will expand, thus changing the periodicity of the grating and in turn the wavelength of light it will back reflect. Alternatively, if a grating is bent, it will change the periodicity of the grating which will change the wavelength of light it will back reflect.

In some embodiments, chirped gratings can be integrated into soft robots by either adding them to the surface of the robots (sealing using an adhesive), or by embedding them inside the soft robots. Embedding could be performed during the molding stage or after molding when several layers of the soft robots are put together. Alternatively, chirped gratings can be integrated or added to a textile that is wrapped around the actuator.

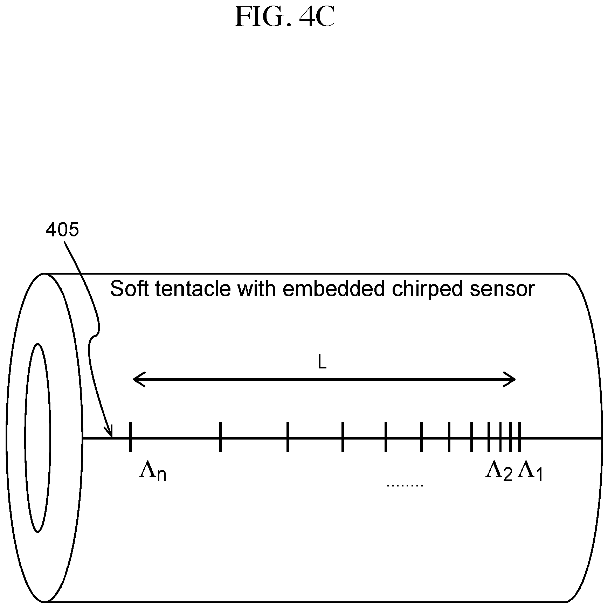

Figure 4C shows a soft tentacle with a chirped grating 405 added to the surface of the tentacle. In other embodiments, the grating-based sensor may be placed in the core of the actuator. The grating's period changes from $\Lambda_1$ to $\Lambda_n$ along the length of the grating (L). Such a grating can detect structural or chemical changes happening along the length of the grating by generating back-reflected modes by each part of the grating. Tracking of the wavelength position or amplitude of the back-reflected modes allows for monitoring of factors affecting mode propagation.

The grating-based sensor may be used in conjunction with one or more of the same type or different sensors. In certain embodiments, the soft robotic device further includes a strain limited layer disposed along one side of the elastomeric body, and the soft robotic device comprises one or more sensors (e.g., grating-based sensors embedded in or attached to the strain limited layer) and one or more sensors (e.g., grating-based sensors) embedded in or attached to the elastomeric body. In some specific embodiments, the soft robotic device further comprises one or more additional sensors each independently selected from the group consisting of grating-based sensor, thermal sensor, chemical sensor, biological analyte sensor, sound sensor, optical sensor, radiological sensor, thermal sensors, strain sensors, chemical sensors, biological sensors, neural sensors, pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, VX gas sensors, sarin gas sensors, mustard gas sensors, explosives detectors, metal detectors, and current sensors.

In some embodiments, the soft robotic device further includes a processor configured to be operably linked to the grating-based sensor to receive the readouts from the grating-based sensor and interpret the readouts; and/or a control system configured to control the movement of the soft robot based on the readouts generated by the grating-based sensor or the processor's interpretation of the readouts. Thus, based on the readings of the sensors such as the grating-based sensor, the control system instructs the robot to take certain actions (e.g., reducing/increasing inlet fluid pressure/flow; avoiding contact with obstacles; moving towards/away from chemical, biological or physical signal sources).

In some embodiments, the state feedback may include:

1): Information on the bending of an actuator. This is relevant, for example, when a bending actuator contacts an object. In this situation, just knowing the inflation pressure of an actuator is insufficient for knowing the degree of bending of the actuator since an object is impeding the motion of the actuator. As a result, a central control computer would need bending data not just pressure data as an input to know how to guide the activity of the soft robot. This feedback loop would be useful in a soft gripper where it is needed to know the bending of the fingers as it grips objects of different shapes. In this situation, the control computer would know when a finger was touching an object by sensing the point when an increase in pressure was not producing an increase in bending. When this point is sensed, the computer could either stop inflating that finger to gently cradle the object it is gripping or continue to inflate that finger in order to apply force to the surface of the object it is gripping.

2): Information on the temperature of the actuator or on the temperature of the ambient. Changes in temperature will change the stiffness of the elastomers that make up a soft actuator. As a result the degree of actuation of an actuator will change as a function of temperature given a fixed actuation pressure. By using temperature data of an actuator as part of a sensing feedback loop a control computer could compensate for this effect to ensure that the desired degree of actuation is always achieved for an actuator, regardless of temperature. This would be relevant for a fire rescue robot that has to walk into hot environments where the parameters for controlling its gate will be constantly changing with temperature. In some embodiments, the sensing of an actuators temperature may be achieved through the use of TFBGs or thermocouples on the actuator or through remote measurements of an actuators temperature using a device such as an infrared thermometer or thermostat.

3): Information about the presence of specific chemical and biological agents. This could be relevant to a soft robot that explores a chemical spill. For example if a silicone-based soft robot finds that one of its legs is in a puddle of chemicals that will damage the robot (e.g. hexane, dioxane, dichloromethane) this information could be fed to the control computer at which point the control computer would work to guide the robot to safety.

4): Pressure sensing would be useful for controlling a soft gripper. For example in the event that one of the fingers of a gripper popped, the pressure sensor could send this information to a controller that begins a corrective action with the remainder of the fingers in order to guarantee that the object being grasped is not dropped and therefore broken, lands on a person, or both. In this scenario, the pressure sensor could be in a variety of places. For example, the sensor could be in the actuator using a Bragg grating or a conventional pressure sensor like a barometric pressure sensor chip or the sensor could be in the box that supplies pressure to the robot via a tether.

5): A flow sensor would be useful for controlling a soft gripper. For example, in the event that one of the fingers of a gripper popped, the flow sensor could send this information to a controller that begins a corrective action with the remainder of the fingers in order to guarantee that the object being grasped is not dropped and therefore broken, lands on a person, or both. The flow sensor could be in a variety of places. For example if a gripper is inflated to a static pressure once the gripper is fully actuated the air flow to the gripper would stop. But if one of the fingers of the gripper popped, the electro pneumatic transducer responsible for maintaining a static pressure in the gripper will start to flow air to the finger to compensate for the pressure drop in the finger due to the pop. As a result this popping of the finger could be measured as an anomalous air flow. At this time the gripper could begin a corrective action. This flow sensor may be in a pneumatic control box controlling the gripper but could also be incorporated into the body of the actuator. Thus, in some embodiments, a soft actuator is described with an embedded flow sensor to monitor changes in the operation flow rate or pressure of the pressurized fluid used as a control input for the actuator so that its operation is modified in response to changes in the fluid flow.

Integration of grating-based sensors with soft robots:

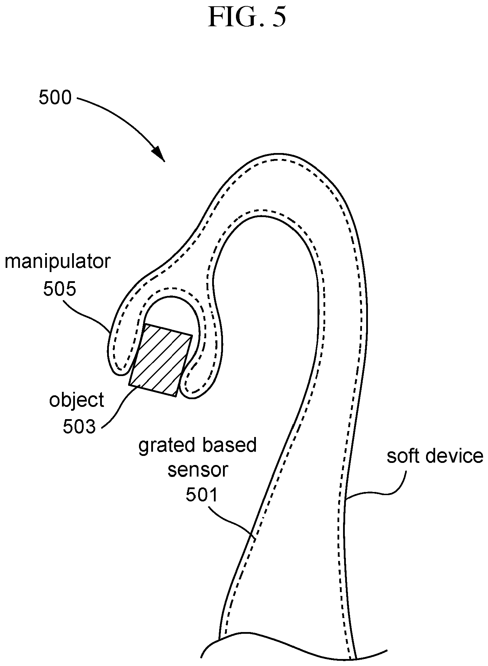

Sensors can be added to a soft robot: 1) Attaching a commercial optical fiber 501 *(e.g.,* SMF-28) with a grating imprinted in its core to the surface of a soft actuator or soft robot **(Figure 5),** and 2) Fabricating custom polymer-based optical waveguides with gratings embedded in their structure that would later be added to the surface of the soft actuator or soft robot, or would be incorporated into the body of a soft actuator or soft robot. Attaching of an optical sensor to the surface of the soft robot could be done with help of any kind of an adhesive that is not going to limit soft robot's and sensor's flexibility. If the sensor has to be integrated inside the robot then sensors could be added in between layers that comprise the body of the robot. In some embodiments, the sensor can also be embedded into the robot during the molding process.

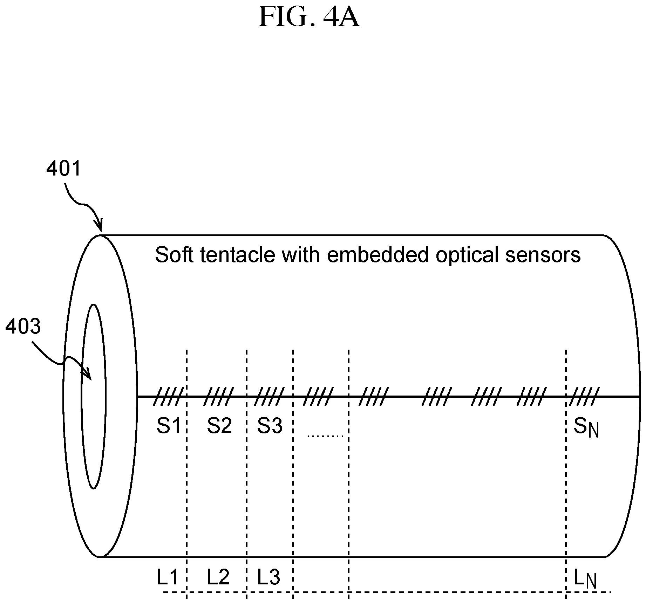

**Figure 4A** shows a schematic diagram showing a soft tentacle 401 with an optical waveguide embedded in its structure. In these embodiments, a plurality of sensors $S_1$, $S_2$ ... $S_n$ are embedded in the soft tentacle. The waveguide has N number of TFBG sensors that are spatially and spectrally separated proving an opportunity to monitor a parameter of interest over the length of the waveguide L=L1+L2+L3+....+Ln. Since the fiber is inextensible, it may be incorporated into the strain limiting layer. This could be done in many different ways. Below is a list of non-limiting examples:

1): In the case of a three-chambered tentacle, the fiber may be molded into the central strain limiting core.

2): In other embodiments, an actuator may be built where the fiber is the strain limiting layer. If a fiber was molded into the wall of an inflatable elastomeric structure upon inflation, the structure would bend in the direction of the inextensible fiber. One can create complex motions using this approach. For example, if the fiber is molded into the wall of an elastomeric tube such that the fiber forms a helix that is winding its way up the wall of the tube upon inflation, the tube would extend and twist.

3) In still other embodiments, a hollow channel may be molded into the strain limiting layer of a soft actuator so that a fiber could be inserted into the channel after molding. The fiber could then be fixed into place by filling the channel with glue or additional elastomer. Alternatively, one could choose not to use glue or more elastomer and just leave the fiber free standing in the channel. One could also use a helical or serpentine shape fiber whose ends are attached to the ends of the channel so the fiber can elongate as the actuator bends, twists, or extends.

4): The fiber could be attached via gluing, sewing, or weaving to a fabric that is then placed on the actuator to act as a strain limiting layer. For example, simple textiles may be used as a strain limiting layer for soft actuators.

In some embodiments, another approached to adding a fiber that would get around the fact that it is inextensible would be to place it into the hollow interior (e.g., hollow space 403 shown in Figure 4A) of an inflation chamber. The fiber could be free standing in the inflation chamber or its ends could be fixed to the walls of the inflation chamber where in this case a fiber that has a helical or serpentine shape can be used so it can elongate as the inflation chamber bends, twists, or extends.

In certain embodiments, if the sensor is meant to measure the bending state of the actuator, it could be placed on the interior or exterior of the actuator. In some embodiments, if it is meant to sense chemical or biological materials, it would need to be either 1) on the exterior of the actuator so that the sensor can come into contact with the external environment or 2) it needs to be located in a hollow tube inside the actuator used to collect samples so the sample flows past the sensor. In some embodiments, if the fiber is meant to measure the inflation pressure of an actuator, the sensor is fixed to an inner wall of an inflation chamber or free standing in the chamber. In some embodiments, if the fiber is being used to measure the temperature of an actuator in order to compensate for changes in the stiffness of the actuator with temperature, the sensors may be installed or embedded at multiple points across the actuator. This is because heat transported in silicone and polyurethane elastomers can take a significant amount of time so one could map the thermal gradients in the device as opposed to taking one temperature measurement at a single point. In some embodiments, if the temperature sensor is used to sense the temperature in the external environment, for example to ensure the robot does not walk into a fire, the sensor may be installed on the exterior of the actuator to ensure that the sensor has a fast response time.

The waveguide has N number of TFBG sensors that are spatially and spectrally separated proving an opportunity to monitor a parameter of interest over the length of the waveguide L=L1+L2+L3+....+Ln. The overall change of the parameter of interest can be found as a sum of sensor responses. Such configurations also allow for the detection of the spatial location of a measured parameter because it can now be spectrally encoded into the response of the sensors.

**Figure 5** illustrates a grating-based sensor 501 integrated into a tentacle arm 500 whereby the sensor 501 can provide state feedback of the arm shape as well as the end effector shape. The sensor can be attached to the surface of the tentacle arm using an adhesive, or it can be embedded into the body of the arm. This could be implemented either by inserting the sensor into the tentacle during the molding process or embedding it in between layers after molding has been performed. Adding of the sensor to the soft robot should be done by integrating the sensor with a strain-limiting layer that would not stretch when a robot is actuated. If the sensor is added to a soft layer that expands upon actuation, then the waveguide should be positioned in a helical, bent, or other spatially modified pattern that would allow for expansion/stretching of the whole integrated structure. Furthermore, deformations in the sensor caused by clamping forces of the gripper/manipulator 505 or collisions with an object 503 in the environment can be detected.

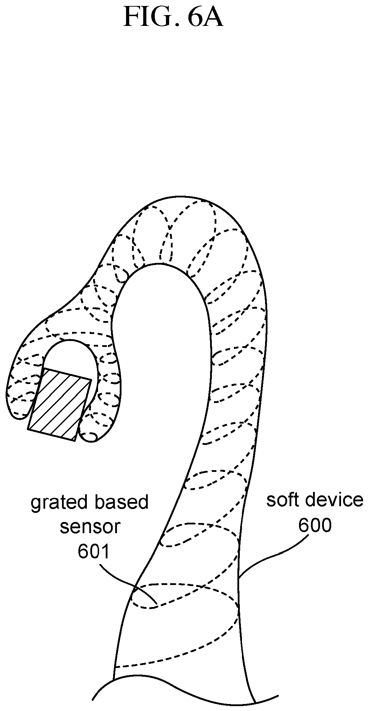

**Figure 6A** illustrates another embodiment of the grating-based sensor 601 in which the sensor 601 is helically wound around the soft device 600. The advantage of this configuration is the sensor minimally impedes the range of motion of the device, there are no moving parts, it provides greater sensory coverage of the soft device, and permits device length extension and contraction.

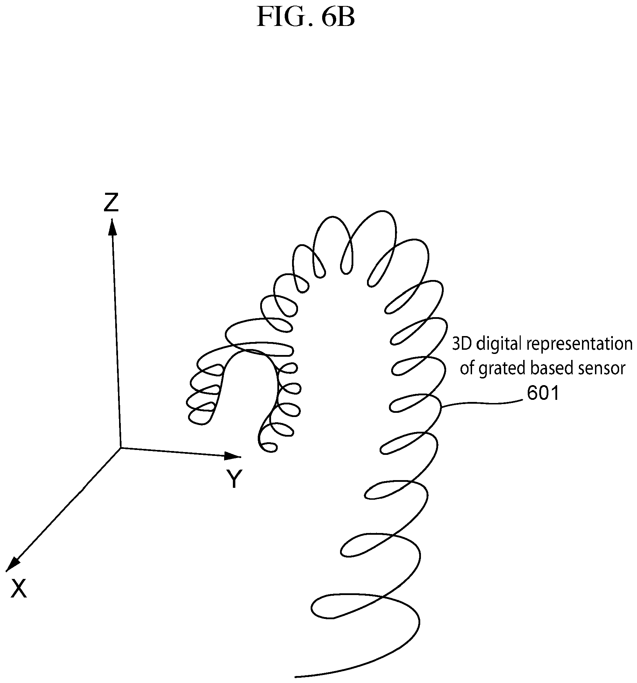

**Figure 6B** illustrates a 3D mapping of the state of the soft device as measured by the grating-based sensor 601. This information can be fed into a controller to program the soft device to execute tasks and collect data about how the device interacts with its environment. For example, if the helix is deformed at an unexpected point, this might suggest a collision with an object.

In some embodiments, it should be noted that the output from this sensor and others (e.g. bump or collision sensors, pressure sensors) could be fed to a haptic device to physically inform the operator the location and intensity of forces acting on the device. For example, the operator could wear a sensorized haptic glove in which a soft robotic device tries to mimic the state of the glove. If the operator squeezes an object via the soft robotic device, contact / pressure sensors in the soft robotic device would activate the relevant haptic actuators in the wearer's glove. This could be used to physically signal to the operator the quality of the grasp or when the soft device contacts an object.

*Soft Robotic Prosthetic Systems*

In another aspect, a soft robotic prosthetic system is described, including: a soft robot configured to assist the movement of one or more muscle or body part of a user and comprising an elastomeric body having one chamber or a plurality of interconnected chambers disposed within the body and a pressurizing inlet that is configured to receive fluid for the chamber or the plurality of interconnected chambers to actuate the soft robot; at least one sensor configured to detect physical, chemical, magnetic, or electronic signal; and at least one of a processor configured to operably linked to the sensor to receive the readouts from the sensor and interpret the readouts; and a control system configured to actuate the soft robot to assist the movement of one or more muscle or body part of a user based on the readouts generated by the one or more sensors or the processor's interpretation of the readouts.

As used herein, prosthesis or a prosthetic device refers to a device, either external or implanted, that substitutes for or supplements a missing or defective part of the body, or generally improves the function/movement of one or more body parts or muscles of a user. Non-limiting examples of users include human and animals. In some embodiments, the soft robotic prosthetic system is attached or otherwise linked to the body of the user and is configured to assist or improve the motion of the body part or muscle of the user. The sensor can be external or internal to the soft robot (e.g., attached or embedded in the soft body or the strain limiting layer). In certain embodiments, the soft robot described herein is a wearable soft robot configured to counteract a body tremor. In certain embodiments, the sensor is configured to measure muscle or neural activity associated with a tremor and the control system is configured to actuate the soft robot in response to counter that tremor.

In some embodiments, the soft robotic prosthetic system is used to act as prosthetics for humans or animals, providing many important tasks such as physical communication, object manipulation, and self-stabilization. To perform these tasks, the soft actuators require signals from a user, the environment, or both. Sensors from the environment may be obtained via one or more sensors described herein. Signals from the user can be generated from a variety of sources, that can broadly be classified as, but are not limited to, i) electrical, ii) magnetic, iii) optical, iv) thermal, v) audible, vi) chemical and vii) mechanical sources. Non-limiting examples of the magnetic signal include signals from magnetoencephalography (MEG) style brain scans.

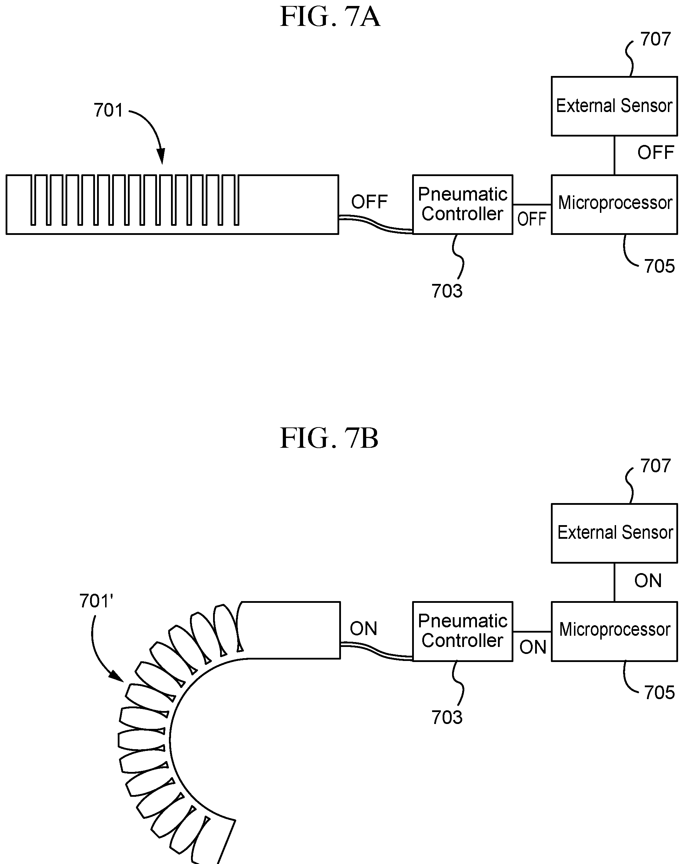

Figures 7A-7B show a soft robotic system containing a soft actuator 701 controlled by a controller system, e.g., a pneumatic controller 703, though hydraulic or vacuum systems could also be used. The pneumatic controller 703 is optionally connected to a microprocessor 707, which dictates whether the pneumatic controller should inflate or deflate the soft actuator. The microprocessor uses signals from an external sensor 707 to determine the state of the pneumatic controller. The external sensor can be driven by any discriminating signal generated by a human or animal body. The signals being transmitted can be done so via wires or by any wireless means. Figures 7A and 7B show the relaxed state (701) and the actuated state (701') of the actuator, respectively.

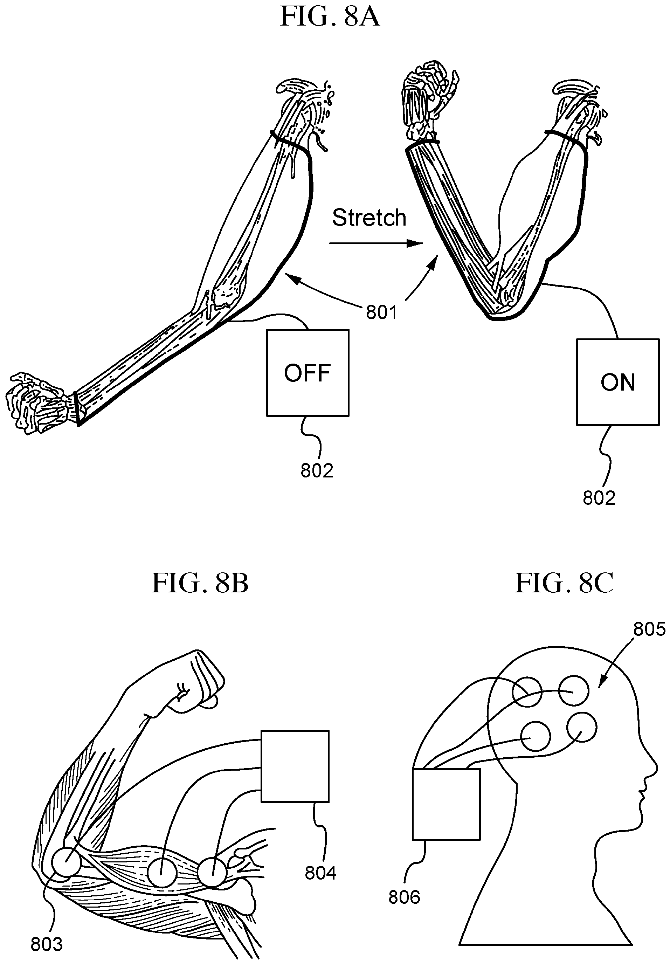

In certain embodiments, the six classes of signals from the user, as described above, can be controlled in three ways: i) by body movement **(Figure 8A),** ii) by muscle excitation **(Figure 8B),** and iii) neuronal excitation **(Figure 8C).** As shown in **Figure 8A**, this could be a strain sensor 801 or system of strain sensors running the length of the arm to indicate arm motion. Here just the sensor is depicted. In some embodiments, a soft actuator runs along the length of the arm as well and the strain sensor is placed on the strain limiting layer of the actuator. In this case when the strain sensor measures the initial arm motion it sends a signal to a controller 802 that begins inflating the soft actuator attached to the arm thereby assisting the arm in its bending motion.

As shown in **Figure 8B,** in certain embodiments, sensors 803 measure electrical signals in the muscle groups of the arm. Here, like in **Figure 8A,** only the sensor is depicted in the figure for simplicity. In some embodiments, a soft actuator described herein is attached to the arm with electrical sensors on its strain limiting layer that are in contact with the surface of the arm. When these sensors measure electrical activity in the muscles of the arm, a controller 804 begins to pressurize the soft actuator which in turn bends the arm to assist the motion of the user.

As shown in **Figure 8C,** sensors 805 are attached to the surface of the head of a user or to the surface of the brain that measure neural activity in the motor cortex. In some embodiments, the sensor is configured to measure the neural activity in the motor cortex associated with the user's intent to move their arm. Based on the readouts of this sensor, a controller 806 may pressurize the soft actuator on the arm thereby assisting the arm to move.

Optically-mediated signals are another form of wireless transmission. For example, LEDs can be placed on the body of a user that emits either visible or infrared light. As the user moves, sensors on the robot or an external visual-recognition device can track changes in the user's movement and change the state of a soft actuator accordingly. Alternatively, reflecting light from an external light source can be processed by the hub, such is done with gaming systems (e.g., Xbox Kinect).

*Soft Robotic Device with Imaging Areas*

In yet another aspect, a soft robotic device is described, including: an elastomeric body having one chamber or a plurality of interconnected chambers disposed within the body and a pressurizing inlet that is configured to receive fluid for the chamber or the plurality of interconnected chambers; and one or more imaging areas configured to provide visual signals different from other areas of the soft robotic device and configured to provide information regarding the state of the soft robotic device.

In some embodiments, at least one of the imaging areas is placed on the surface of the elastomeric body or embedded inside the elastomeric body. In other embodiments, the soft robotic device further comprises a strain limited layer disposed along one side of the elastomeric body; and at least one of the imaging areas is on the surface of the strain limited layer or embedded inside the strain limited layer. In still other embodiments, the soft robotic device may have one or more imaging areas placed on the surface of the elastomeric body or embedded inside the elastomeric body and one or more imaging areas on the surface of the strain limited layer or embedded inside the strain limited layer.

In some embodiments, the imaging areas are colored areas, e.g., colored marks, having a color different from other areas of the soft robotic device. The color may be recognizable by the naked eye or a motion detecting system configured to track and/or detect the colored area.

Thus, in some embodiments, one or more colored marks are applied to the surface of a soft robotic device, e.g., the surface of the elastomeric body, to aid motion tracking by a computer vision system. Marks of any colors may be used. These marks provide reference points along the body of the soft robot that can be distinguished by the vision system for determining the state of the soft robot. Since the surface of a soft robot strains during actuation, these tracking color marks will change shape, area, and color intensity. As a result, in addition to being able to use these marks to determine the position, velocity, acceleration, orientation, momentum, etc. at points along the actuator, it will also be possible to determine the stress state, strain state, and/or morphology at these points by analyzing these changes in shape, area, and color intensity.

In other embodiments, the soft robotic device further comprises a strain limited layer disposed along one side of the elastomeric body; and at least one of the imaging areas is on the surface of the strain limited layer or embedded inside the strain limited layer. In some specific embodiments, at least one of the imaging areas is a colored area having a color different from other areas of the soft robotic device and the soft robotic device further comprises a motion detecting system configured to track and/or detect the colored area. Thus, in these embodiments, because the strain limiting layer does not deform substantially, the colored area may substantially retain their shape, area, and color intensity and the colored area can be used as position indicators. This configuration may simplify the interpretation of the images captured by a motion capture system and reduce the complexity of computer calculations.

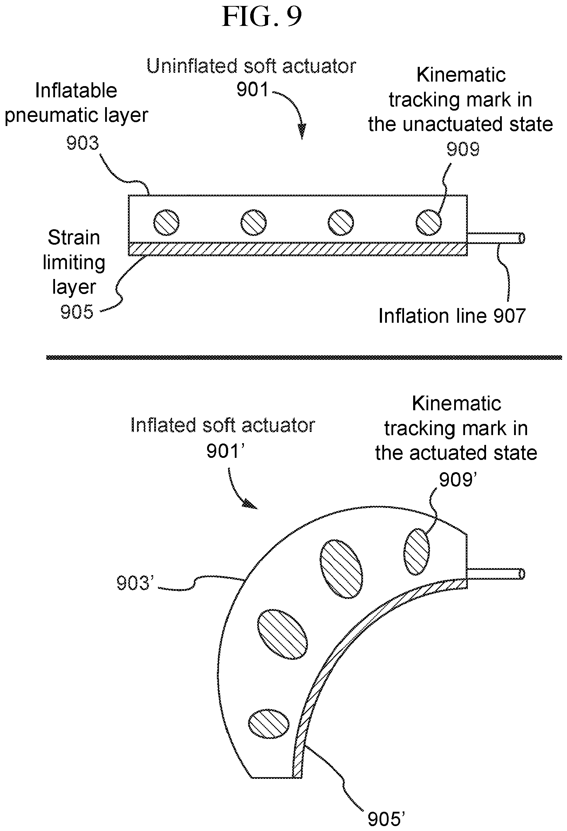

As shown in **Figure 9**, several imaging areas, *e.g.,* colored marks 909 are applied onto the surface of the inflatable pneumatic layer 903 of a soft actuator 901. The soft actuator 901 includes the inflatable pneumatic layer 903, inflation line 907, and a strain limiting layer 905 in contact with or attached to the inflatable pneumatic layer 903 (top portion of **Figure 9** shows the soft actuator in an uninflated state). During inflation (bottom portion of **Figure 9),** the strain limiting layer bends (905'), the inflatable pneumatic layer expands (903') and the areas of the tracking marks (909') increase; and their shape changes and their color density decreases. Such information may be captured by an imaging device for analysis. The imaging device may, for example, compare the color densities of the tracking marks at the uninflated stage with those of the inflated stage, and decreases in the color density indicate that the soft robot is in a strained or actuated state. Similarly, the imaging device may compare the areas of the tracking marks at the uninflated stage with those of the inflated stage, and increases in the areas indicate that the soft robot is in a strained or actuated state.

One of the main reasons that conventional roboticist use kinematic tracking marks, is that identifying these marks using a computer vision system is easier than having a computer visually recognize the body of the robot itself. Using this approach for tracking the motion of a soft robot can be difficult because, unlike the colored marks on a hard robot, these marks on soft robots will change shape, area, and color intensity during actuation. In certain embodiments, the imaging areas, the kinematic tracking marks, are included in the strain limiting layer of a soft actuator. Since the strain limiting layer is the section of a soft actuator that experiences the least strain, the kinematic tracking marks on this layer will display the smallest degree of shape, area, and color intensity change during actuation thereby simplifying the process of recognizing and analyzing these marks using a computer vision system.

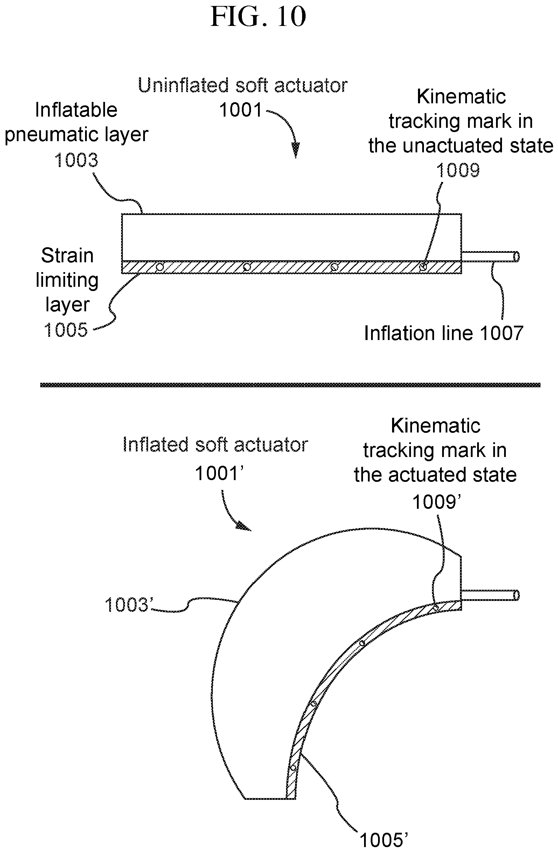

These embodiments are described herein with reference to **Figure 10,** where several imaging areas, *e.g.,* colored marks 1009, are applied onto the surface of the strain limiting layer 1005 of a soft actuator 1001. The soft actuator 1001 includes the inflatable pneumatic layer 1003, inflation line 1007, and a strain limiting layer 1005 in contact with or attached to the inflatable pneumatic layer 1003 (top portion of **Figure 10** shows the soft robot in an uninflated state). During inflation, the soft actuator is actuated (see 1001'), the inflatable pneumatic layer expands (see 1003'), the strain limiting layer bends (see 1005'), and the tracking marks (1009')' relative positions changes but the area and color densities of the tracking marks may remain unchanged or only change minimally (e.g., less than about 10%, 8%, 5%, 4%, 2% or 1%, or the change is in a range bounded by any percentages disclosed herein). Such information may be captured by an imaging device for analysis. The imaging device may compare the positions of the tracking marks at the uninflated stage with those of the inflated stage, and a change in their relative positions indicates that the soft robot is in a strained or actuated state.

In yet another embodiment, the imaging area is a mark embedded in or attached to the surface of the soft actuator consisting of a patch of radiocontrast, e.g., a chemical which is recognizable via an imaging device, *e.g.,* a medical imaging device. In certain embodiments, the imaging system is a PET scan imaging system. Any known chemical used in medical imaging can be used. In certain embodiments, the chemical is a barium salt such as barium sulfate. In some embodiments, a soft robotic medical device may include patches of radiocontrast (e.g., barium sulfate) in the straining sections of the body of the robot for the determination of the position, velocity, acceleration, orientation, momentum and strain/morphology at points along the actuator using an X-ray imaging system, e.g., a CT (X-ray computed tomography) imaging system or a fluoroscope imaging system. In other embodiments, the radiocontrast material comprises a MRI dye and the imaging device comprises a MRI. In certain embodiments, a pure patch of radiocontrast can be located in a void or pocket inside the actuator or the contrast can be mixed into the elastomer used to construct the actuator. In certain embodiments, the soft robot, including a patch of radiocontrast, can be used at a location not visible to the user. For example, a soft robotic tentacle can be used in the abdomen during laparoscopic surgery or a colonoscopy.

In some embodiments, the soft robotic device further includes one or more additional sensors each independently selected from the group consisting of grating-based sensor, thermal sensor, chemical sensor, biological analyte sensor, sound sensor, optical sensor, radiological sensor, thermal sensors, strain sensors, chemical sensors, biological sensors, neural sensors, pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, VX gas sensors, sarin gas sensors, mustard gas sensors, explosives detectors, metal detectors, and current sensors.

In some other embodiments, the soft robot further includes at least one of a motion-tracking system configured to detect the imaging area and an imaging device configured to detect the imaging area; and a control system configured to control the movement of the soft robot based on the readouts generated by the motion-tracking system or the imaging device.

### Soft Robotic System having Distributed sensor networks:

In a further aspect, a soft robotic system is described, including: a soft robot comprising an elastomeric body having one chamber or a plurality of interconnected chambers disposed within the body and a pressurizing inlet that is configured to receive fluid for the chamber or the plurality of interconnected chambers; a network of sensors for sensing a signal; and a processor operably linked to the network of sensors and configured to determine the location, gradient, and/or presence of a signal based on the sensors' readouts.

In some embodiments, the processor in the soft robotic system may include a suitable algorithm to calculate the location of the signal. In some embodiments, the soft robotic system further includes a control system configured to control the movement of the soft robot based on the readouts generated by the one or more sensors or the processor's interpretation of the readouts. The control system may be configured to control the soft robot to move towards or away from the location of the signal.

In some embodiments, the soft robotic system further comprises a strain limited layer disposed along one side of the elastomeric body; and at least one of the sensors is on the surface of the strain limited layer or embedded inside the strain limited layer. In other embodiments, at least one of the sensors is on the surface of the elastomeric body or embedded inside the elastomeric body.

In some embodiments, the signals to be detected include, but are not limited to, light, sound, heat, radioactive materials, chemicals, biologicals, electric fields and magnetic fields. The correspondingly suitable sensor may be used.

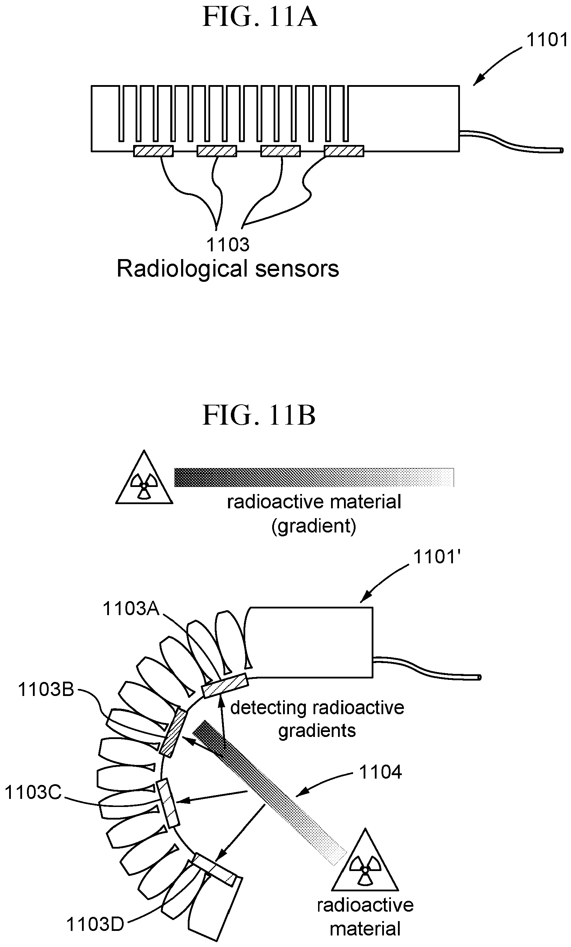

In certain embodiments, a spatially distributed network of sensors is used for determining the direction of the source of a signal and/or the location of the source of a signal. In certain embodiments, one or more radiological sensors are included in the soft actuator. One embodiment is shown in **Figure 11A,** where a plurality of radiological sensors 1103 is included in soft robot 1101. As shown in **Figure 11B,** the soft robot is in its actuated state 1101' and placed close to a radioactive material gradient 1104 where the denser color indicates more concentration of the radioactive material. Radiological sensor 1103B is closest to the most concentrated area of the radioactive material and thus will produce the strongest signal (as indicated by the darkest color bar). The concentration of the radioactive material decreases in the order of the areas close to sensor 1103A, to 1103C, to 1103D. And thus the signals produced by these sensors will decrease in the same order (1103A > 1103C > 1103D, as shown by the darkness of their relative color bar in **Figure 11B).** The processor in the soft robotic system may include a suitable algorithm to calculate the location of the signal.

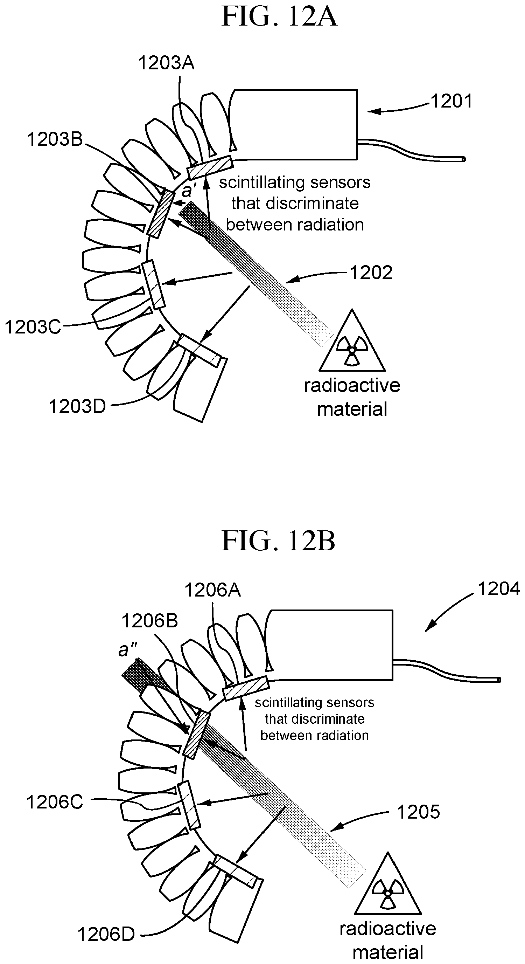

As shown in **Figure 12A,** a soft robot 1201 with scintillating sensors 1203A-D is used to detect a radioactive material gradient 1202. Sensors 1203A and 1203B are closest to the strongest concentration of the radioactive material (as indicated by the darkest color bar) and thus will produce the strongest scintillation. Sensor 1203C will produce stronger scintillation signal than sensor 1203D, due to its location relative to the radioactive material gradient. **Figure 12B** shows a situation where the soft robot 1204's body overlaps with the radio material gradient 1205. In this case, the scintillation signal decreases in the order of scintillating radio material sensor 1206B, 1206A, 1206C, and 1206D, due to their relative locations to the radio material gradient 1205.

Note that **Figures 12A** and **12B** demonstrates the detection of the radioactive source at different location. Thus, the relative distance between the individual sensors and the points along the radiological gradient are different in **Figures 12A** and **12B.** In **Figure 12B,** the signal measured across the network will be different than in **Figure 12A.** For example in **Figure 12A,** sensor 1203B is almost on top of the peak of the gradient (shown by arrow $\alpha'$) but in **Figure 12B,** sensor 1203B is further away (shown by arrow $\alpha''$). This information alone may be enough to estimate the distance between sensor 1203B and the source of the radiation but not the direction of the source.

With the help of the other sensors, one can figure out the direction as well as the distance to the source of the radiation. For instance, the difference in signals of sensor 1203B in **Figures 12A and 12B** may enable one to conclude that the radioactive source has moved. If the source moves down and to the right in **Figure 12B,** one would expect to see a rise in signals on sensors 1206C and 1206D. However, in the embodiments as shown, in **Figure 12B** the source moves up and to the left. As a result, one would see that sensor 1206D will have a large drop in signal relative to what sensor 1203D measures in **Figure 12A.** Similarly, one would expect to see that there is a drop on sensor 1206C's signal in **Figure 12B.** As a result, one may determine the direction of the signal by using a plurality of (e.g., two, three, or more) spatially distributed sensors.

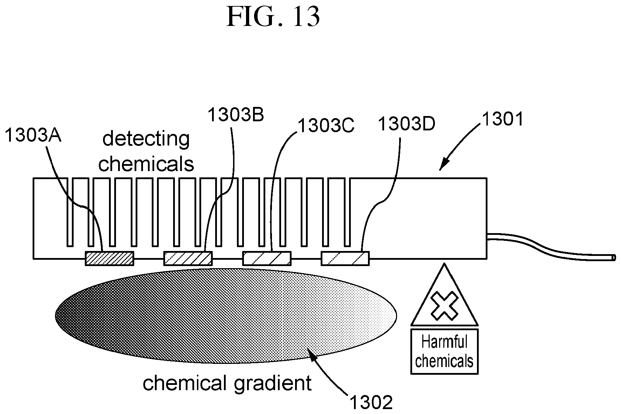

In certain embodiments, one or more chemical sensors (1303A-D) are embedded in or attached to the surface of the soft robot or soft actuator 1301 **(Figure 13)** to detect the presence of a harmful chemical gradient 1302. A chemical sensor is a device that transforms chemical information, ranging from the concentration of a specific sample component to total composition analysis, into an analytically useful signal. The chemical information may originate from a chemical reaction of the analyte or from a physical property of the system investigated. A range of conventional hard electronic chemical sensors such as the ethanol sensors used in breathalyzers, flexible chemiresistors based on conductive polymers (e.g., Polythiophenes), or carbon nanotubes mixed with sensitizing chemicals can be used. Alternatively, one can suspend conductive particles in an elastomer (making the elastomer conductive) and measure the change in resistance of the elastomer as it swells due to coming in contact with a chemical of interest. In the case shown in **Figure 13,** due to their relative position to the chemical gradient 1302 (darker color indicates an area with more chemical concentration), the strength of the signal decreases in the order of sensor 1303A > 1303D > 1303C > 1303D. Based on the readouts of these sensors, the processor may generally determine the location of the chemical signal, i.e., to the left side of the Figure.

In certain embodiments, chemical weapons sensors or chemical sensors can be applied at multiple points along the body of a soft robot and connected, via wires (e.g., lithographically patterned serpentine wires), to a central processing unit (a processor). This processing unit would then analyze the relative intensity of the chemical signal being measured by different sensors across the soft robot. By combining this information with the knowledge of the relative location of each sensor, the direction from which the chemical signal originated is determined. Without wishing to be bound by any particular theory, it is believed that if one sensor is closer to the location of the chemical spill, it will measure a larger signal. Thus, by using three or more sensors, one may estimate/determine the chemical signal gradient in three dimensional spaces and therefore get a better estimate of the location of the signal. Alternatively, an algorithm may be used by a computer to mathematically fit the sensor data to determine the location and/or gradient of the chemical signal.

Information culled from a distributed network of sensors could be used to guide the actions of a soft robot. For example, this network of chemical weapons sensors can provide information for a controller system to guide a soft surveillance robot to the location of a chemical agent of interest. In a non-limiting example, a soft robot with a plurality of chemical (e.g., VX) sensors (e.g., a sensor array) is described. In Step 1), the sensors in the array would take a measurement of the chemical reading. In Step 2), a processor of the soft robot would then identify the sensor with the highest signal and the sensor with the lowest signal in the sensor array. In Step 3), the processor would define the straight line path from the sensor with the lowest signal to the sensor with the highest signal as the direction of the gradient. In Step 4), the processor would then command the robot to walk along that straight line path in the direction of increasing signal for a fixed distance (e.g., 1 meter). Step 5), the soft robot sensors will take another measurement of the signals in the sensor array and repeat the process. By iterating these steps, the soft robot will eventually move to the source of the chemical source.

In one or more embodiments, the sensor is a biological sensor configured to provide an electrode pair or a plurality of electrodes and related circuitry, such as is suitable for conducting an immune assay or detect the presence and concentration of various analytes such as, but are not limited to, glucose, urea, ion concentrations, heavy metals, lactate, uric acid and the like. In a non-limiting embodiment, the sensor is a glucose sensor and the soft robot comprises a test area and an electrode for interaction with a meter mounted on the soft robot device, e.g., strain limited layer.

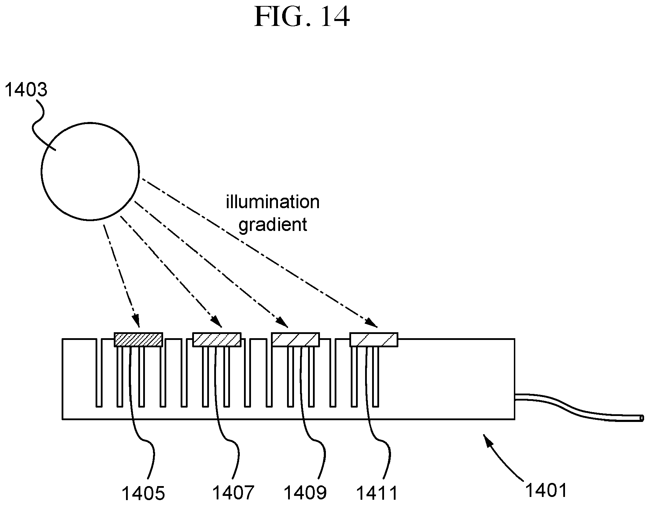

Shown in **Figure 14** is a soft robot 1401 including a plurality of optical sensors 1405, 1407, 1409, 1411. Due to their relative distance from the illumination source 1403, the strength of the signal decreases in the order of sensor 1405 > 1407 > 1409 > 1411. Based on the readouts of these sensors, the processor may generally determine the location of the illumination source.

In some embodiments, the sensor is a temperature sensor. The temperature sensor may be embedded in the strain limiting layer or the pneumatic layer of the soft robot or soft actuator. In other embodiments, the temperature sensor may be attached to the surface of the strain limiting layer or the pneumatic layer of the soft robot or soft actuator. In certain embodiments, the temperature sensor is included inside the pneumatic layer to measure the temperature of the gas or fluid inside the pneumatic layer.

Any temperature sensor known in the art can be used. Non-limiting examples of temperature sensors include thermistor, resistive temperature detector, and thermocouple.

The mechanical properties of an elastomeric material, such as stiffness, are strongly correlated with temperature. Changes in temperature can reversibly or permanently alter the physical behavior of soft actuators. A temperature sensor, embedded in or attached to the soft actuator, can detect changes in the working temperature of the elastomeric materials used in the construction of the actuator and a microprocessor based control system can make adjustments to the fluid pressures used to actuate the actuator to compensate for the changes in the mechanical properties of the elastomers. For example since the stiffness of elastomers change with temperature, a soft actuator will require a different inflation pressure to achieve a given actuated shape at different temperatures. In certain embodiments, a control system is designed to use temperature data in order to assure that a soft actuator inflates to the same shape regardless of its temperature by modulating the actuation pressure as needed.

In certain embodiments, one may measure the temperature inside the actuator to determine if the temperature is outside of the safe working range of the elastomers that make up the actuator thereby triggering the shutdown of the robotic system. For example if the temperature of the actuator goes below a certain threshold (typically below -100C for silicones) the elastomer will become embrittled. As a result inflating the actuator could result in the rupture of the actuator destroying the robot.

In yet another aspect, a soft robotic system is described, including: a soft robot comprising an elastomeric body having one chamber or a plurality of interconnected chambers disposed within the body and a pressurizing inlet that is configured to receive fluid for the chamber or the plurality of interconnected chambers; one or more thermal sensors; and a processor operably linked to one or more thermal sensors and configured to control the fluid pressurization of the chambers based on the thermal sensors' readouts.

In certain embodiments, at least one of the thermal sensors is embedded or attached to the elastomeric body of the soft robot. In other embodiments, the soft robot further includes a strain limited layer disposed along one side of the elastomeric body; and at least one of the thermal sensors is attached to the surface of the strain limited layer or embedded inside the strain limited layer. In other embodiments, at least one of the thermal sensors is located at a distance away from the soft robot. The thermal sensor may be used to measure the temperature of the environment immediately close to the soft robot or the environment remote from the soft robot. At least one of the thermal sensors may be located about 0.1 m, 0.3 m, 0.5 m, 1 m, 5 m, 10 m, 50 m, 100 m, 200 m, 500 m, or 1000 m away from the soft robot, or in a range bounded by any two values disclosed herein.

Thus, in certain embodiments, the processor is configured to interpret the readout from the thermal sensor to perform real time measurement or estimation of the soft robotic device's stiffness and/or morphology based on the temperature readings from the thermal sensors. In turn, the processor will control the fluid pressurization of the chambers to compensate for the temperature of the actuator and/or the surrounding environment. That is, if the elastomeric body is stiffer under colder temperature, the processor will increase the fluid pressure or fluid volume inside the chambers to ensure that the desired inflation state/morphology is achieved. On the other hand, if the elastomeric body is less stiff under hotter temperature, the processor will decrease the fluid pressure inside the chambers to ensure that the desired inflation state/morphology is achieved.

In certain embodiments, the processor is linked to the thermal sensors by wire connection or Wi-Fi to receive the thermal sensors' readouts. Based on the readouts, the processor may control the fluid pressurization of the chamber, e.g., through a fluid pump, to adjust the fluid amount inside the chamber.

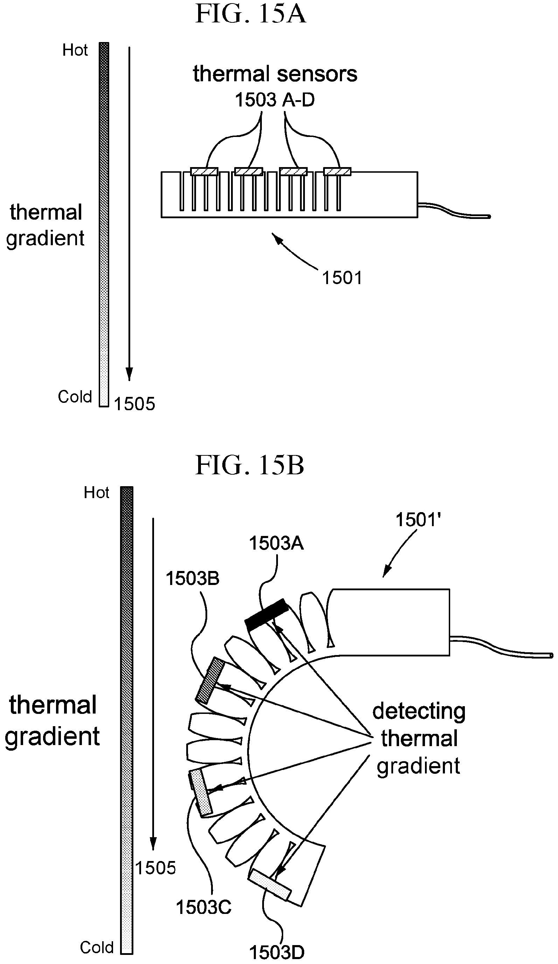

Shown in **Figure 15A** is a soft robot 1501 including a plurality of thermal sensors 1503A-D to detect a vertical thermal gradient indicated by arrow 1505 (temperature decreases from the top of the **Figure 15A** to the bottom of the **Figure 15A).** Because theses sensors are essentially at the same vertical thermal gradient, sensors 1503A-D will generate the same or similar temperature readings. However, in **Figure 15B,** the soft robot is actuated (shown as 1501'), the sensors will reside in different locations with respect to the vertical thermal gradient. As a result, the temperature readings generated by the sensor will decrease in the order of 1503A > 1503B > 1503C > 1503D. Based on the different readings of the sensors in the actuated and unactuated state of the robot, the process may deduce the direction of the thermal gradient based on a computer algorithm.

In certain specific embodiments, the temperature sensor is a thermocouple configured to provide a voltage measurement and the voltage is correlated to a temperature of the strain limited layer or the elastomeric body. In other embodiments, the temperature sensor is a resistance temperature detector, thermistor, or zener diode, and resistance or voltage is measured for temperature determination. In certain embodiments, the elastomer's stiffness as a function of temperature is known, so one may determine the stiffness of the elastomer based on the temperature readout and in turn determine the curvature of the actuator as a function of inflation pressure using finite element analysis to achieve a temperature dependent calibration method. In other embodiments, one can inflate the actuator at different temperatures and measure its curvature as a function of pressure to develop a calibration method empirically.

Since the surface of a soft actuator strains during actuation, the relative distance between sensors will not remain fixed. This change in the relative distance between sensors in the network will complicate the determination of the direction of a signal of interest. To minimize this issue, in certain embodiments, a spatially distributed network of sensors could be applied to the strain limiting layer (e.g., embedded in or attached to the surface of the strain limiting layer) of a soft robot since the strain limiting layer is the section of the soft robot that experiences the least strain during actuation.

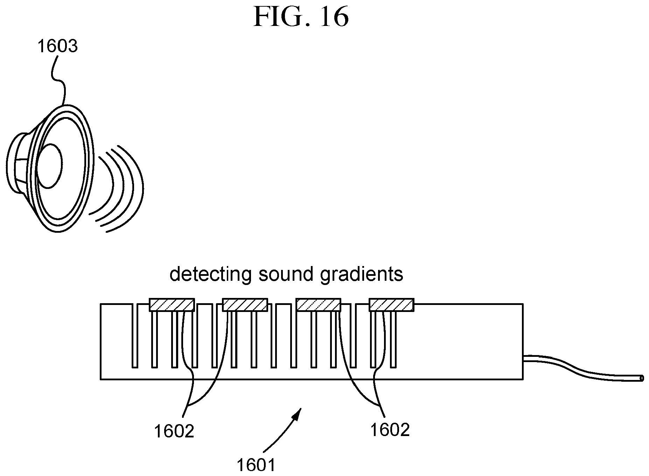

In certain embodiments, the sensor is a sound sensor. As shown in **Figure 16,** a plurality of sound sensors 1602 is attached on the surface of a soft robot 1601. Because of the difference in distances from each sound sensor to the sound source 1603, the readouts from the sensors are different. Based on these differences, the processor may include an algorithm to deduce or calculate the location of the sound source.

In certain embodiments, a system is described, including the soft robot described according to any embodiment disclosed herein, and at least one of a processor and a control system controlling the movement of the robot. In certain embodiments, based on the information obtained by the sensors (e.g., various radioactive material sensor, chemical sensor, sound sensor, or illumination sensor, or any sensor described herein), a user and/or processor can use the readouts to estimate the location of the source of the signal (e.g., radioactive material, chemical, the sound source, or the illumination source). Furthermore, if the soft robot is included in a system comprising a controller controlling the movement of the soft robot, the controller may control the soft robot to move closer to the source of the signal to confirm the location of the source (the signal obtained by the sensor will generally become stronger if the source location estimation is correct) and/or to further investigate the source. For instance, the soft robot may further include one or more position sensors to determine the relative position of the robot in relation to the signal source and the controller may use this information to guide the movement of the robot to move closer or away from the signal source.

In certain embodiments, the soft robot has an embedded or attached position sensor such as a GPS unit that is configured to determine the soft robot's absolute location. In these embodiments, the soft robot could combine the knowledge of its absolute location (from the GPS unit) with its estimation of the relative location of a signal source (any of the signal sources described herein that are being measured with a sensor array or network) to provide an estimation of the absolute location of the signal source. For example, if the soft robot comprises a GPS unit and an array of chemical sensors (e.g., VX sensors), it can determine the relative distance between itself and the VX from the array of VX sensors and treat that as an offset from the absolute location of the robot from the GPS sensor. The final value from this calculation would be the absolute location of the source of the VX. This information could then be transmitted to a user at a remote location.

In yet another aspect, a method for sensing the state of the soft robotic device of any one of the embodiments disclosed herein is described, including obtaining readouts from the one or more sensors or imaging areas; and determining a state of the soft robotic device.

Unless otherwise defined, used or characterized herein, terms that are used herein (including technical and scientific terms) are to be interpreted as having a meaning that is consistent with their accepted meaning in the context of the relevant art and are not to be interpreted in an idealized or overly formal sense unless expressly so defined herein. For example, if a particular composition is referenced, the composition may be substantially, though not perfectly pure, as practical and imperfect realities may apply; e.g., the potential presence of at least trace impurities (e.g., at less than 1% or 2%) can be understood as being within the scope of the description; likewise, if a particular shape is referenced, the shape is intended to include imperfect variations from ideal shapes, e.g., due to manufacturing tolerances. Percentages or concentrations expressed herein can represent either by weight or by volume.

Although the terms, first, second, third, etc., may be used herein to describe various elements, these elements are not to be limited by these terms. These terms are simply used to distinguish one element from another. Thus, a first element, discussed below, could be termed a second element without departing from the teachings of the exemplary

EP 3 245 028 B1

embodiments. Spatially relative terms, such as "above," "below," "left," "right," "in front," "behind," and the like, may be used herein for ease of description to describe the relationship of one element to another element, as illustrated in the figures. It will be understood that the spatially relative terms, as well as the illustrated configurations, are intended to encompass different orientations of the apparatus in use or operation in addition to the orientations described herein and depicted in the figures. For example, if the apparatus in the figures is turned over, elements described as "below" or "beneath" other elements or features would then be oriented "above" the other elements or features. Thus, the exemplary term, "above," may encompass both an orientation of above and below. The apparatus may be otherwise oriented (e.g., rotated 90 degrees or at other orientations) and the spatially relative descriptors used herein interpreted accordingly. Further still, in this disclosure, when an element is referred to as being "on," "connected to," "coupled to," "in contact with," etc., another element, it may be directly on, connected to, coupled to, or in contact with the other element or intervening elements may be present unless otherwise specified.

The terminology used herein is for the purpose of describing particular embodiments and is not intended to be limiting of exemplary embodiments. As used herein, singular forms, such as "a" and "an," are intended to include the plural forms as well, unless the context indicates otherwise.

It will be appreciated that while a particular sequence of steps has been shown and described for purposes of explanation, the sequence may be varied in certain respects, or the steps may be combined, while still obtaining the desired configuration. Additionally, modifications to the disclosed embodiment and the invention as claimed are possible and within the scope of this disclosed invention as defined in the appended claims.

**Claims**

1. A soft robotic device comprising:

    an elastomeric body having one chamber or a plurality of interconnected chambers disposed within the body and a pressurizing inlet that is configured to receive fluid for the chamber or the plurality of interconnected chambers;
    a strain limited layer (905) disposed along one side of the elastomeric body; and
    at least one fiber Bragg grating-based optical sensor.

2. The soft robotic device of claim 1, wherein the grating-based sensor is configured to detect a physical, chemical, biological, or electronic signal; or

    wherein the grating-based sensor is selected from the group consisting of tilted fiber Bragg gratings sensor, chirped gratings sensor, and long period Bragg gratings sensor; or wherein the grating-based sensor is configured to provide information regarding the state of the soft robotic device; or
    wherein the grating-based sensor is configured to provide information regarding the state of the soft robotic device and wherein the state of the soft robotic device is selected from the group consisting of the pressure, temperature, position, length, curvature, orientation, velocity, acceleration, morphology, stress, strain, and physical state at points along the soft robotic device.

3. The soft robotic device of claim 1, wherein the grating-based sensor is configured to provide information regarding the external environment of the soft robotic device.

4. The soft robotic device of any one of the preceding claims, wherein the grating-based sensor is configured to detect temperature, humidity, chemical agent or biological agent in the external environment of the soft robotic device; or the grating-based sensor is configured to detect strain, force, magnetic field, flow, bending, directional bending, three-dimensional state, vibration, pressure, temperature information of the soft robot.

5. The soft robotic device of any one of the preceding claims, wherein the grating-based sensor is embedded in the elastomeric body or attached to the outside of the elastomeric body.

6. The soft robotic device of claim 5, wherein the grating-based sensor is molded or co-molded into the elastomeric body.

7. The soft robotic device of claim 5, where the grating-based sensor is sewn, glued, or snapped on to the elastomeric body or secured to the elastomeric body with hook and loop.

8. The soft robotic device of claim 6, wherein the grating-based sensor is removable from the elastomeric body; or

wherein the grating-based sensor helically winds around the elastomeric body or part thereof.

9. The soft robotic device of any one of the preceding claims, wherein the soft robotic device comprises one or more grating-based sensors embedded in or attached to the strain limited layer (905).

10. The soft robotic device of any one of the preceding claims, wherein the soft robotic device further comprises a plurality of spectrally separated grating-based sensors with different periods.

11. The soft robotic device of claim 10, wherein the plurality of spectrally separated grating-based sensors with different periods are disposed together along the length of a single piece of fiber or disposed individually and spliced together.

12. The soft robotic device of any one of the preceding claims, wherein the pressurizing inlet is configured to receive fluid from an external fluid source.

13. The soft robotic device of any one of the preceding claims, wherein the soft robotic device comprises one or more grating-based sensors embedded in or attached to the strain limited layer and one or more grating-based sensors embedded in or attached to the elastomeric body.

14. The soft robotic device of any one of the preceding claims, wherein the soft robotic device further comprises one or more additional sensors each independently selected from the group consisting of grating-based sensor, biological analyte sensor, sound sensor (1602), optical sensor (1405), radiological sensor (1103), thermal sensors (1503), strain sensors (801), chemical sensors (1303), biological sensors, neural sensors (805), pressure sensors, barometric pressure sensors, vacuum sensors, altimeters, conductivity sensors, impedance sensors, inertial measurement units, force sensing resistors, laser range finders, acoustic range finders, magnetometers, Hall Effect sensors, magneto-diodes, magneto-transistors, MEMS magnetic field sensors, microphones, photo detectors, accelerometers, gyroscope sensors, flow sensors, humidity sensors, chemiresistors, volatile organic compound sensors, heavy metal sensors, pH sensors, sedimentation sensors, cardiac ablation sensors, myoelectric sensors, electronic noses, gas sensors, oxygen sensors, nitrogen sensors, natural gas sensors, chemical weapons sensors, VX gas sensors, sarin gas sensors, mustard gas sensors, explosives detectors, metal detectors, and current sensors.

15. The soft robotic device of any one of the preceding claims, further comprising at least one of a processor configured to operably linked to the grating-based sensor to receive the readouts from the grating-based sensor and interpret the readouts; and a control system configured to control the movement of the soft robot based on the readouts generated by the grating-based sensor or the processor's interpretation of the readouts.


**Patentansprüche**

1. Weichrobotervorrichtung, umfassend:

   einen Elastomerkörper, der eine Kammer oder eine Vielzahl von miteinander verbundenen Kammern, die innerhalb des Körpers angeordnet sind, und einen Druckbeaufschlagungseinlass aufweist, der konfiguriert ist, um Fluid für die Kammer oder die Vielzahl von miteinander verbundenen Kammern zu empfangen;
   eine dehnungsbegrenzte Schicht (905), die entlang einer Seite des Elastomerkörpers angeordnet ist; und
   zumindest einen faseroptischen gitterbasierten Bragg-Sensor.

2. Weichrobotervorrichtung nach Anspruch 1, wobei der gitterbasierte Sensor konfiguriert ist, um ein physikalisches, chemisches, biologisches oder elektronisches Signal zu erfassen; oder

   wobei der gitterbasierte Sensor aus der Gruppe ausgewählt ist, die aus geneigtem Faser-Bragg-Gitter-Sensor, Chirp-Gitter-Sensor und Bragg-Gitter-Sensor mit langer Periode besteht; oder
   wobei der gitterbasierte Sensor konfiguriert ist, um Informationen bezüglich des Zustands der Weichrobotervorrichtung bereitzustellen; oder
   wobei der gitterbasierte Sensor konfiguriert ist, um Informationen bezüglich des Zustands der Weichrobotervorrichtung bereitzustellen, und wobei der Zustand der Weichrobotervorrichtung aus der Gruppe ausgewählt ist, die aus dem Druck, der Temperatur, der Position, der Länge, der Krümmung, der Ausrichtung, der Geschwindigkeit, der Beschleunigung, der Morphologie, der Spannung, der Dehnung und dem physikalischen Zustand an Punkten entlang der Weichrobotervorrichtung besteht.

3. Weichrobotervorrichtung nach Anspruch 1, wobei der gitterbasierte Sensor konfiguriert ist, um Informationen bezüglich der äußeren Umgebung der Weichrobotervorrichtung bereitzustellen.

4. Weichrobotervorrichtung nach einem der vorhergehenden Ansprüche, wobei der gitterbasierte Sensor konfiguriert ist, um Temperatur, Feuchtigkeit, chemisches Mittel oder biologisches Mittel in der äußeren Umgebung der Weichrobotervorrichtung zu erfassen; oder der gitterbasierte Sensor konfiguriert ist, um Informationen zu Dehnung, Kraft, Magnetfeld, Durchfluss, Biegung, Richtungsbiegung, dreidimensionalem Zustand, Vibration, Druck, Temperatur des Weichroboters zu erfassen.

5. Weichrobotervorrichtung nach einem der vorhergehenden Ansprüche, wobei der gitterbasierte Sensor in den Elastomerkörper eingebettet oder an der Außenseite des Elastomerkörpers angebracht ist.

6. Weichrobotervorrichtung nach Anspruch 5, wobei der gitterbasierte Sensor in den Elastomerkörper eingeformt oder angeformt ist.

7. Weichrobotervorrichtung nach Anspruch 5, wobei der gitterbasierte Sensor auf den Elastomerkörper genäht, geklebt oder eingerastet ist oder mit Klettverschluss an dem Elastomerkörper befestigt ist.

8. Weichrobotervorrichtung nach Anspruch 6, wobei der gitterbasierte Sensor von dem Elastomerkörper entfernbar ist; oder
wobei sich der gitterbasierte Sensor spiralförmig um den Elastomerkörper oder einen Teil davon windet.

9. Weichrobotervorrichtung nach einem der vorhergehenden Ansprüche, wobei die Weichrobotervorrichtung einen oder mehrere gitterbasierte Sensoren umfasst, die in die dehnungsbegrenzte Schicht (905) eingebettet oder daran angebracht sind.

10. Weichrobotervorrichtung nach einem der vorhergehenden Ansprüche, wobei die Weichrobotervorrichtung ferner eine Vielzahl von spektral getrennten gitterbasierten Sensoren mit unterschiedlichen Perioden umfasst.

11. Weichrobotervorrichtung nach Anspruch 10, wobei die Vielzahl von spektral getrennten gitterbasierten Sensoren mit unterschiedlichen Perioden zusammen entlang der Länge eines einzelnen Faserstücks angeordnet oder einzeln angeordnet und zusammengespleißt sind.

12. Weichrobotervorrichtung nach einem der vorhergehenden Ansprüche, wobei der Druckbeaufschlagungseinlass konfiguriert ist, um Fluid von einer externen Fluidquelle zu empfangen.

13. Weichrobotervorrichtung nach einem der vorhergehenden Ansprüche, wobei die Weichrobotervorrichtung einen oder mehrere gitterbasierte Sensoren, die in die dehnungsbegrenzte Schicht eingebettet oder daran angebracht sind, und einen oder mehrere gitterbasierte Sensoren umfasst, die in den Elastomerkörper eingebettet oder daran angebracht sind.

14. Weichrobotervorrichtung nach einem der vorhergehenden Ansprüche, wobei die Weichrobotervorrichtung ferner einen oder mehrere zusätzliche Sensoren umfasst, die jeweils unabhängig aus der Gruppe ausgewählt sind, die aus gitterbasiertem Sensor, biologischem Analytsensor, Schallsensor (1602), optischem Sensor (1405), radiologischem Sensor (1103), Wärmesensoren (1503), Dehnungssensoren (801), chemischen Sensoren (1303), biologischen Sensoren, neuronalen Sensoren (805), Drucksensoren, Luftdrucksensoren, Vakuumsensoren, Höhenmessern, Leitfähigkeitssensoren, Impedanzsensoren, Trägheitsmesseinheiten, Kraftmesswiderständen, Laserentfernungsmessern, akustischen Entfernungsmessern, Magnetometern, Hall-Effekt-Sensoren, Magnetdioden, Magnettransistoren, MEMS-Magnetfeldsensoren, Mikrofonen, Fotodetektoren, Beschleunigungsmessern, Gyroskopsensoren, Durchflusssensoren, Feuchtigkeitssensoren, Chemiresistoren, Sensoren für flüchtige organische Verbindungen, Schwermetallsensoren, pH-Sensoren, Sedimentationssensoren, Herzablationssensoren, myoelektrischen Sensoren, elektronischen Nasen, Gassensoren, Sauerstoffsensoren, Stickstoffsensoren, Erdgassensoren, Sensoren für chemische Waffen, VX-Gassensoren, Saringassensoren, Senfgassensoren, Sprengstoffdetektoren, Metalldetektoren und Stromsensoren besteht.

15. Weichrobotervorrichtung nach einem der vorhergehenden Ansprüche, ferner umfassend zumindest eines von einem Prozessor, der konfiguriert ist, um mit dem gitterbasierten Sensor wirkverbunden zu sein, um die Auslesungen von dem gitterbasierten Sensor zu empfangen und die Auslesungen zu interpretieren; und einem Steuersystem, das konfiguriert ist, um die Bewegung des Weichroboters basierend auf den Auslesungen, die durch den gitterbasierten Sensor erzeugt werden, oder der Interpretation der Auslesungen durch den Prozessor zu steuern.

**Revendications**

1. Dispositif robotique souple comprenant :

   un corps élastomère comportant une chambre ou une pluralité de chambres interconnectées disposées à l'intérieur du corps et une entrée de pressurisation qui est conçue pour recevoir un fluide pour la chambre ou la pluralité de chambres interconnectées ;
   une couche de déformation limitée (905) disposée le long d'un côté du corps élastomère ; et
   au moins un capteur optique à base de fibre à réseau de Bragg.

2. Dispositif robotique souple selon la revendication 1, ledit capteur à base de réseau étant conçu pour détecter un signal physique, chimique, biologique ou électronique ; ou

   ledit capteur à base de réseau étant sélectionné dans le groupe constitué d'un capteur à fibre à réseaux de Bragg inclinés, d'un capteur à réseaux comprimés et d'un capteur à réseaux de Bragg à longue période ; ou
   ledit capteur à base de réseau étant conçu pour fournir des informations concernant l'état du dispositif robotique souple ; ou
   ledit capteur à base de réseau étant conçu pour fournir des informations concernant l'état du dispositif robotique souple et ledit état du dispositif robotique souple étant sélectionné dans le groupe constitué de la pression, la température, la position, la longueur, la courbure, l'orientation, la vitesse, l'accélération, la morphologie, la contrainte, la déformation et l'état physique au niveau de points le long du dispositif robotique souple.

3. Dispositif robotique souple selon la revendication 1, ledit capteur à base de réseau étant conçu pour fournir des informations concernant l'environnement externe du dispositif robotique souple.

4. Dispositif robotique souple selon l'une quelconque des revendications précédentes, ledit capteur à base de réseau étant conçu pour détecter la température, l'humidité, un agent chimique ou un agent biologique dans l'environnement externe du dispositif robotique souple ; ou
   ledit capteur à base de réseau étant conçu pour détecter des informations de déformation, de force, de champ magnétique, d'écoulement, de flexion, de flexion directionnelle, d'état tridimensionnel, de vibration, de pression et de température du robot souple.

5. Dispositif robotique souple selon l'une quelconque des revendications précédentes, ledit capteur à base de réseau étant intégré dans le corps élastomère ou fixé à l'extérieur du corps

6. Dispositif robotique souple selon la revendication 5, ledit capteur à base de réseau étant moulé ou co-moulé dans le corps élastomère.

7. Dispositif robotique souple selon la revendication 5, ledit capteur à base de réseau étant cousu, collé ou encliqueté sur le corps élastomère ou fixé au corps élastomère avec un crochet et une boucle.

8. Dispositif robotique souple selon la revendication 6, ledit capteur à base de réseau étant amovible du corps élastomère ; ou
   ledit capteur à base de réseau s'enroulant en hélice autour du corps élastomère ou d'une partie de celui-ci.

9. Dispositif robotique souple selon l'une quelconque des revendications précédentes, le dispositif robotique souple comprenant un ou plusieurs capteurs à base de réseau intégrés dans la couche de déformation limitée (905) ou fixés à celle-ci.

10. Dispositif robotique souple selon l'une quelconque des revendications précédentes, ledit dispositif robotique souple comprenant en outre une pluralité de capteurs à base de réseau séparés spectralement avec des périodes différentes.

11. Dispositif robotique souple selon la revendication 10, ladite pluralité de capteurs à base de réseau séparés spectralement avec des périodes différentes étant disposés ensemble sur la longueur d'un seul tronçon de fibre ou disposés individuellement et reliés ensemble.

12. Dispositif robotique souple selon l'une quelconque des revendications précédentes, ladite entrée de pressurisation étant conçue pour recevoir un fluide en provenance d'une source de fluide externe.

13. Dispositif robotique souple selon l'une quelconque des revendications précédentes, ledit dispositif robotique souple comprenant un ou plusieurs capteurs à base de réseau intégrés dans la couche de déformation limitée ou fixés à celle-ci et un ou plusieurs capteurs à base de réseau intégrés dans le corps élastomère ou fixés à celui-ci.

14. Dispositif robotique souple selon l'une quelconque des revendications précédentes, ledit dispositif robotique souple comprenant en outre un ou plusieurs capteurs supplémentaires, chacun sélectionné indépendamment dans le groupe constitué d'un capteur à base de réseau, d'un capteur de substance à analyser biologique, d'un capteur sonore (1602), d'un capteur optique (1405), d'un capteur radiologique (1103), de capteurs thermiques (1503), de capteurs de déformation (801), de capteurs chimiques (1303), de capteurs biologiques, de capteurs neuronaux (805), de capteurs de pression, de capteurs de pression barométrique, de capteurs de vide, d'altimètres, de capteurs de conductivité, de capteurs d'impédance, d'unités de mesure inertielle, de résistances de détection de force, de télémètres laser, de télémètres acoustiques, de magnétomètres, de capteurs à effet Hall, de magnéto-diodes, de magnéto-transistors, de capteurs de champ magnétique MEMS, de microphones, de photodétecteurs, d'accéléromètres, de capteurs gyroscopiques, de capteurs d'écoulement, de capteurs d'humidité, de résistances chimiques, de capteurs de composés organiques volatils, de capteurs de métaux lourds, de capteurs de pH, de capteurs de sédimentation, de capteurs d'ablation cardiaque, de capteurs myoélectriques, de nez électroniques, de capteurs de gaz, de capteurs d'oxygène, de capteurs d'azote, de capteurs de gaz naturel, de capteurs d'armes chimiques, de capteurs de gaz VX, de capteurs de gaz sarin, de capteurs de gaz moutarde, de détecteurs d'explosifs, de détecteurs de métaux et de capteurs de courant.

15. Dispositif robotique souple selon l'une quelconque des revendications précédentes, comprenant en outre au moins l'un d'un processeur configuré pour être lié de manière fonctionnelle au capteur à base de réseau pour recevoir les lectures en provenance du capteur à base de réseau et interpréter les lectures ; et un système de commande conçu pour commander le mouvement du robot souple sur la base des lectures générées par le capteur à base de réseau ou de l'interprétation des lectures par le processeur.

# FIG. 1

Optical fiber $\Lambda$

$\lambda$ $\lambda_B$

$\lambda - \lambda_B$

Incident light

Transmitted light

# FIG. 2

$\lambda_{clad}$ $\alpha$ $\Lambda$ Optical fiber

$\lambda_B$

$\lambda$

Transmitted light

# FIG. 3

$\lambda_{B1}$ $\lambda_{B2}$ $\lambda_{Bn}$

$\text{TFBG}_1 \text{TFBG}_2$ $\text{TFBG}_n$ Optical fiber

$\lambda$

Reflected light

Transmitted light

# FIG. 4A

401

403

Soft tentacle with embedded optical sensors

S1  S2  S3  ........  $S_N$

L1  L2  L3  $L_N$

FIG. 4B

# FIG. 4C

405

Soft tentacle with embedded chirped sensor

L

$\Lambda_n$ ........ $\Lambda_2$ $\Lambda_1$

# FIG. 5

500

manipulator
505

object
503

grated based
sensor
501

soft device

# FIG. 6A

grated based
sensor
601

soft device
600

# FIG. 6B

3D digital representation
of grated based sensor
601

## FIG. 7A

701

External Sensor 707

OFF

Microprocessor 705

OFF Pneumatic Controller 703

OFF

## FIG. 7B

701'

External Sensor 707

ON

Microprocessor 705

ON Pneumatic Controller 703

ON

# FIG. 8A

Stretch

801

OFF

ON

802

802

# FIG. 8B

803

804

# FIG. 8C

805

806

# FIG. 9

Uninflated soft actuator
901

Inflatable
pneumatic layer
903

Kinematic
tracking mark in
the unactuated state
909

Strain
limiting
layer
905

Inflation line 907

Inflated soft actuator
901'

Kinematic
tracking mark in
the actuated state
909'

903'

905'

# FIG. 10

Uninflated soft actuator
1001

Inflatable
pneumatic layer
1003

Kinematic
tracking mark in
the unactuated state
1009

Strain
limiting
layer
1005

Inflation line 1007

Inflated soft actuator
1001'

Kinematic
tracking mark in
the actuated state
1009'

1003'

1005'

# FIG. 11A

1101

1103
Radiological sensors

# FIG. 11B

radioactive material
(gradient)

1101'

1103A

1103B

detecting radioactive
gradients

1103C

1104

1103D

radioactive
material

# FIG. 12A

1203A

1203B

1201

scintillating sensors
that discriminate
between radiation

a'

1202

1203C

1203D

radioactive
material

# FIG. 12B

1206A

1206B

1204

a''

scintillating sensors
that discriminate
between radiation

1206C

1205

1206D

radioactive
material

# FIG. 13

# FIG. 14

# FIG. 15A

thermal sensors
1503 A-D

Hot

thermal
gradient

Cold ⌷ 1505

1501

# FIG. 15B

Hot

1503B    1503A    1501'

thermal
gradient

detecting
thermal
gradient

1505

1503C

Cold ⌷

1503D

## FIG. 16

1603

detecting sound gradients

1602

1602

1601

## REFERENCES CITED IN THE DESCRIPTION

**Patent documents cited in the description**

- US 20120271339 A1
- US 20140180134 A1
- US 3981528 A
- WO 2012148472 A
- US 1328250 W
- US 1322593 W
- US 61885092